(19) Europäisches Patentamt
European Patent Office
Office européen des brevets

(11) Publication number: **0 428 708 B1**

(12) **EUROPEAN PATENT SPECIFICATION**

(45) Date of publication of patent specification: **05.10.94**

(51) Int. Cl.5: **C08F 16/32**

(21) Application number: **90909909.5**

(22) Date of filing: **04.06.90**

(86) International application number:
**PCT/US90/03151**

(87) International publication number:
**WO 90/15082 (13.12.90 90/28)**

(54) **PROCESS FOR PREPARING A POLYMER HAVING PERFLUOROCYCLOBUTANE RINGS AND POLYMERS CONTAINING PERFLUOROCYCLOBUTANE RINGS.**

(30) Priority: **09.06.89 US 364667**
**15.12.89 US 451404**

(43) Date of publication of application:
**29.05.91 Bulletin 91/22**

(45) Publication of the grant of the patent:
**05.10.94 Bulletin 94/40**

(84) Designated Contracting States:
**AT BE CH DE ES FR GB IT LI NL SE**

(56) References cited:
**EP-A- 0 303 292**
**GB-A- 1 126 554**
**US-A- 3 418 302**

(73) Proprietor: **THE DOW CHEMICAL COMPANY**
**2030 Dow Center,**
**Abbott Road**
**Midland, MI 48640 (US)**

(72) Inventor: **BABB, David, A.**
**419 Narcissus**
**Lake Jackson, TX 77566 (US)**
Inventor: **CLEMENT, Katherine, S.**
**126 Daisy**
**Lake Jackson, TX 77566 (US)**
Inventor: **RICHEY, W., Frank**
**316 Linden Lane**
**Lake Jackson, TX 77566 (US)**
Inventor: **EZZELL, Bobby, R.**
**1310 Ashly Court**
**Midland, MI 48640 (US)**

(74) Representative: **Sternagel, Hans-Günther, Dr. et al**
**Patentanwälte Dr. Michael Hann**
**Dr. H.-G. Sternagel**
**Sander Aue 30**
**D-51465 Bergisch Gladbach (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

This invention relates to polymers having a backbone comprising aromatic hydrocarbyl groups, perfluorocyclobutane rings and non-carbon atoms.

It has long been recognized that perfluorovinyl compounds having more carbon atoms than tetrafluoroethylene are very difficult to polymerize into aliphatic chains. Such difficulties are discussed, for instance, in in U S-A-2,848,504 and 2,958,685; and in J. Polymer Science, Part 1-A, pp. 481-492 (1952) and vol. 6, pp 711-717 (1968).

Dimerization of certain perfluorovinyl compounds has been reported and is discussed, for instance, in Chambers, Fluorine in Organic Chemistry, John Wiley, New York, 1973, pp. 173-191; S. Patai, The Chemistry of Alkenes, Wiley Interscience Publishers, 1964, p. 779; M. Hudlicky, Chemistry of Organic Fluorine Compounds, 2nd ed., Halsted Press (John Wiley and Sons), 1972, p. 450; and Tarrant ed., Fluorine Chemistry Reviews, Vol. 2, Marcel Dekker, 1968 pp. 1-52. In general, the dimerizations are easily sterically hindered and have not been used to prepare long chain molecules. U S-A-3,316,312 describes dimerization linking two molecules of such compounds as perfluoropropylene and perfluoropentene-1, and speculates that the reaction could be used for perfluoroalkyl perfluorovinyl compounds wherein the alkyl radical has 1 to 20, "or even a higher number" of carbon atoms.

Such dimerization has not previously been reported to produce compounds having more than one perfluorocyclobutane ring. In fact, few compounds having multiple perfluorinated four carbon-rings have been reported. U S-A-3,303,145 discloses a number of polyethers formed from cyclic fluorocarbon epoxides, which polymers can have perfluorocyclobutane rings separated by oxygen atoms. The polymers are said to have good thermal stability and chemical inertness as well as dielectric properties. Use as solvents, heat-transfer media and lubricants as well as insulators in the form of films and moldings is suggested. U S-A-3,682,876 discloses polyperfluorocyclobutene and halogen terminated polyperfluorocyolobutadienes. The solid polyperfluorocyclobutadienes are reported to be thermally stable, chemically inert and useful as coatings, ablatives, gaskets, bearings, potting compounds and sealants. U S-A-3,900,380 discloses polymers prepared by coupling certain perfluoroalkyl or perfluoroalkyl ether chains with iodine terminated perfluorocyclobutanes to prepare polymers having double bonds suitable for cross linking. The liquids are reported to be useful as hydraulic fluids, and the solids as gaskets and ablatives. Certain polymers containing radical-initiated rings, assumed for steric reasons to be four-membered rings are reported by Brown et al. in J. Polymer Sci: Part A vol. 3, pp 1641-1660 (1965) ad vol. 4, pp 131-140 (1966). None of these reported polymers having perfluorocyclobutane rings is formed by thermal reaction of perfluorovinyl groups. Also, none has aromatic structure.

GB-A-1,126,554 discloses the polymerization of perfluorodivinyl ethers to produce oligomers comprising perfluorocyclobutane rings. The oligomers are prepared by heating the monomers at a temperature of from 100 °C to 200 °C at high pressure, preferably in the presence of a free radical polymerization inhibitor. The oligomeric syrups as well as the monomeric perfluorodivinyl ethers are polymerized by a free radical mechanism resulting in a highly crosslinked hard and tough thermoset resin.

It is the object of the present invention to provide polymers containing perfluorocyclobutane rings that have wide ranges of utility and are easy to prepare and a process for preparing such polymers. The polymers should be solids, fluids or gels having good physical properties and good processability.

The object is solved by a polymer having a backbone comprising aromatic hydrocarbyl groups, perfluorocyclobutane rings and non-carbon atoms.

The invention further relates to a process for preparing a polymer having a backbone comprising aromatic hydrocarbyl groups, perfluorocyclobutane rings and non-carbon atoms by:

(a) contacting monomers having at least two dimerizable perfluorovinyl groups represented by Formula I:

$$CF_2 = CF\text{-}X\text{-}R\text{-}(X\text{-}CF = CF_2)_m$$

wherein R represents an unsubstituted or inertly substituted aromatic hydrocarbyl group, each X is any group which links R and a perfluorovinyl group, and m is an integer of from 1 to 3; and

(b) exposing the monomers to sufficient heat of from 40 °C to 450 °C such that a polymer containing perfluorocyclobutane rings is formed thermally.

In another aspect the invention relates to a process for preparing a polymer having a backbone comprising aromatic hydrocarbyl groups, perfluorocyclobutane rings and non-carbon atoms by:

(a) contacting monomers having two dimerizable perfluorovinyl groups;

(b) exposing the monomers to sufficient heat of from 50 °C to 400 °C such that a polymer containing perfluorocyclobutane rings is formed thermally, and

(c) exposing the polymer to sufficient heat from 100 °C to 450 °C such that crosslinking occurs and a crosslinked polymer is produced.

The crosslinked polymers are advantageously elastomeric in the range from their glass transition temperatures to the temperatures at which degradation is observed, which is generally on the order of 400 °C to 450 °C. Compared to their thermoplastic counterparts, the crosslinked polymers exhibit enhanced solvent resistance and increased mechanical strength, without loss of advantageous electrical properties, such as low dielectric constant and dissipation factor.

Polymers of the invention are formed by thermal reaction of monomers having at least two dimerizable perfluorovinyl groups such that perfluorocyclobutane groups are formed. A dimerizable perfluorovinyl group is a perfluorovinyl group which reacts with another such group to form a perfluorocyclobutane ring. Thus, resulting polymers have at least two perfluorocyclobutane groups, in particular sufficient perfluorocyclobutane groups to achieve physical and electrical properties desired for specific uses of the polymers. The term "polymer" is used herein to refer to any compound having at least two perfluorocyclobutane groups formed from perfluorovinyl groups, and includes oligomers which have from 2 to 100 repeating units and preferably have a molecular weight of from 300 to 30,000. It is within the scope of the present invention to form lower molecular weight oligomers useful as fluids or prepolymers and higher molecular weight polymers exhibiting general plastic properties. Within this scope and depending on the molecular structure connecting the perfluorocyclobutyl groups, the number of perfluorocyclobutane groups can vary from as few as two up to thousands. The process of forming polymers or oligomers by the process of the present invention is general and capable of forming products having wide ranges of utility. Physical and electrical properties of the resulting products are highly dependent on the choice of the molecular structure between the perfluorocyclobutane groups as well as the number of perfluorocyclobutane groups.

The relative proportion by weight of the perfluorocyclobutane groups to the other molecular components of the resulting products can vary over a wide range of from 12 to 1 to 0.01 to 1, preferably from 5 to 1 to 0.02 to 1 and most preferably from 2 to 1 to 0.03 to 1. High proportions of perfluorocyclobutane groups are desirable for instance, when fluorocarbon character such as low dielectric constant is beneficial in the products. Exemplary of such products are low dielectric fluids and lubricants. Medium ranges of ratios of weights of perfluorocyclobutane groups to other molecular structures of 2 to 1 to 1 to 4 are desirable, for instance, when higher physical strength and relatively lower dielectric constants (for example, relative to conventional engineering thermoplastics) are desired, for example, in low dielectric plastics. These relatively low dielectric plastics are particularly preferred and are preferably achieved by using aromatic compounds substituted with trifluorovinyl groups, most preferably, with trifluorovinyl ether groups. Very low proportions of the perfluorocyclobutane groups result, for instance, when low molecular weight oligomers (for example, in the range of 1000 to 20,000) are terminated by trifluorovinyl groups and then thermally dimerized to form higher molecular weight polymers.

Any monomer having at least two dimerizable perfluorovinyl groups is suitably used in the practice of the present invention. Whereas polyaddition of perfluorovinyl groups to form perfluoroaliphatic polymers (like polytetrafluoroethylene), not generally having perfluorocyclobutane groups, takes place in the presence of free radicals or free radical generating catalysts, dimerization to form perfluorocyclobutane groups takes place thermally.

In the thermal polymerization of diperfluorovinyl compounds, substantially linear polymers having little branching are believed to be formed. In the practice of one embodiment of the present invention, certain of these substantially linear polymers are crosslinked. Crosslinking involves chemical reactions that interconnect polymer molecules. As these reactions proceed, a polymer network is formed. Early in a crosslinking process, there are molecules having a wide variety of molecular weights; molecular weight increases with increasing extent of crosslinking. At a point in the progress of crosslinking, the gel point is reached. This point is defined as the point when there is sufficient crosslinking that the polymer is no longer soluble in a solvent for the corresponding uncrosslinked linear polymer. Rather, the polymer swells in the solvent. Theoretically, either the weight average molecular weight diverges to infinity in an infinite sample, or a first macromolecular cluster grows to be on the order of the sample size when the sample is finite. At the gel point, the polymer system loses its solubility and a steady-shear viscosity approaches infinity. In the practice of the present invention at least two types of crosslinking are observed. A first type of crosslinking involves the use of monomers having at least three perfluorovinyl groups such crosslinking is referred to herein as "polyfunctional crosslinking." A second type of crosslinking is observed when certain types of monomers are used, and because it is believed that this second type of crosslinking involves certain aromatic structures in the backbone of the polymer, it is referred to herein as "backbone crosslinking." In the case of backbone crosslinking, a decrease in percent elongation as measured by the procedure of

ASTM D882-83 is also observed. Preferably, the decrease in percent elongation is at least about 10 percent, more preferably at least about 20 percent of the percent elongation. At the gel point, there are still unattached polymer molecules within a polymer network system. As these molecules are crosslinked into the network, stiffness increases and the mechanical strength of the polymer (for example, as measured by the procedures of ASTM D882-83 and ASTM D790-81) is enhanced. The viscosity also continues to increase. The gel point at a temperature can be determined rheologically by the process of H. H. Winter et al. in J. Rheology, 30(2), 367-382 (1986) and 31(8), 683-697, (1987); and Macromolecules, 22, 411-414, (1989). As measured by the procedure taught by Winters, crosslinked polymers of this invention preferably have gel points within about two hours at about 360°C, more preferably in less than about two hours at 320°C, most preferably in less than about two hours at 280°C. Measurements at temperatures below about 320°C are more indicative of a preferred crosslinking because crosslinking at such temperatures is accompanied by thermal decomposition.

Before crosslinking, solid polymers of the invention are generally thermoplastic. Viscosity of either a melt or solution of the polymer increases as crosslinking occurs until the gel point and resulting unsolubility is reached. Backbone crosslinked polymers are preferably elastomeric, that is, the polymer can generally regain its shape after deformation. That deformation is indicated by elongation measurements greater than about 100 percent at temperatures above the glass transition temperature (Tg) of the polymer. Backbone crosslinked polymers preferably retain their elastomeric properties at temperatures of from their glass transition temperatures to the temperatures at which they are observed to degrade, preferably about 400°C. The glass transition temperature varies with the composition of the polymer.

Backbone crosslinking also increases a polymer's tensile strength as measured by the procedures of ASTM D882-83. The increase is preferably up to 1000 percent, more preferably from 10 percent to 500 percent, most preferably of from 10 percent to 100 percent increase. Also the polymer's tensile and flexural modulus as measured by the procedures of ASTM D882-83 and ASTM 0790-81, respectively, also increases, preferably up to 1000 percent, more preferably of from 10 percent to 500 percent, most preferably of from 10 percent to 100 percent. Additionally, the fluorine-containing structures of such crosslinked polymers preferably retain relatively low dielectric constants.

Although any monomer having too dimerizable perfluorovinyl groups and which is crosslinkable is suitably used for backbone crosslinking, backbone crosslinked polymers of the invention are preferably prepared from monomers having two perfluorovinyl groups separated by at least one hydrocarbyl group having at least one carbon atom between the perfluorovinyl groups.

When a perfluorovinyl group is dimerizable, dimerization is preferably favored over other thermal reactions either kinetically or in equilibrium. In perfluorobutadiene, isomerization and formation of perfluorocyclobutane rings is favored; it is, therefore, preferable in the practice of the present invention that the perfluorovinyl groups on a monomer used in the practice of the present invention be separated by at least one atom or group of atoms, which group does not facilitate isomerization. The atom or group of atoms preferably includes at least one carbon atom, more preferably at least one carbon atom in an, optionally substituted, hydrocarbyl group, that is a group containing at least one carbon-hydrogen bond, for instance, a methylene group, a phenylene group, a phenylene ether group or a pyridinyl group.

Furthermore, when the perfluorovinyl groups are attached to aliphatic carbons or separated from aliphatic carbons by single atoms such as oxygen, the perfluorovinyl groups are preferably primary or secondary because tertiary perfluorovinyl groups are generally sterically hindered with respect to formation of perfluorocyclobutane rings, more preferably the perfluorovinyl groups are primary because secondary perfluorovinyl groups tend to rearrange. To avoid rearrangement and facilitate polymer formation and crosslinking the monomers have structures such that resulting polymers have aromatic hydrocarbyl groups, perfluorocyolobutane rings and at least one non-carbon atom such as oxygen, silicon, boron, phosphorus, nitrogen, selenium, tellurium and/or sulfur atom (each optionally substituted) in the backbones.

The monomers preferably have a structure represented by the following Formula I:

Formula I $CF_2=CF\text{-}X\text{-}R\text{-}(X\text{-}CF=CF_2)_m$

wherein R represents an optionally inertly substituted aromatic hydrocarbyl group; each X is independently a bond or any group which links R and a perfluorovinyl group (hereinafter linking structures), said structures being inert; m+1 is the number of $-X-CF=CF_2$ units. m is an integer of from 1 to 3, preferably from 1 to 2. While compounds represented by Formula I wherein m is one are especially useful for forming linear polymers, compounds wherein m is 2 or more particularly 2 or 3 are especially useful for polyfunctional crosslinking. By "inert" it is meant that the structures or substituents do not react undesirably with perfluorovinyl groups or interfere undesirably with polymerization (perfluorocyclobutane formation) of the

monomers.

Linking structures X are each independently a linking structure such as a bond, an oxygen atom, carboxylic and thiocarboxylic ester groups, other sulfur containing structures, perfluoroalkylene, perfluoroalkylene ether, alkylene, acetylene, phosphorus containing groups such as phosphines, carbonyl and thiocarbonyl groups; seleno; telluro; nitrido; silicon-containing groups such as silanediyl, trisilanediyl tetrasilanetetrayl, siloxanediyl, disiloxanediyl, trisiloxyl, trisilazanyl, or silylthio groups; boron-containing groups such as boranediyl or methylboranediyl groups; a combination thereof, or any other group which is inert, which molecularly links R to a perfluorovinyl group, and which provides a molecular structure in which the perfluorovinyl group is sufficiently reactive to form a perfluorocyclobutane ring. For instance, X is preferably other than a perfluoroalkylene group because perfluorovinyl groups attached to perfluoroalkylene groups generally require temperatures greater than about 300 °C to dimerize and are subject to isomerization.

It is preferred that at least one of X is not a bond. More preferably, X is independently selected from the group consisting of groups having at least one non carbon atom between the perfluorovinyl groups and R, such as groups containing oxygen, sulfur, selenium atoms, tellurium atoms, silicon, boron, phosphorus or nitrogen between R and the perfluorovinyl group, for example, oxygen atoms, sulfur atoms, (thio) carboxylic ester groups, phosphines, (thio) carbonyl groups, seleno, telluro, silanediyl, trisilanediyl, trisilazanyl or silylthio, boranediyl groups. Preferred groups have S, O, Si, N or P, more preferably S, O, or Si between R and the perfluorovinyl group, such as carbonyl, thiocarbonyl, sulfone, sulfoxy, silanediyl, amines (optionally inertly substituted), oxygen or sulfur atoms. Most preferably there is a single atom other than carbon between R and each perfluorovinyl group; even more preferably the single atom is oxygen or sulfur, among those groups preferably an ether or sulfide linkage, because monomers having such linking structures advantageously form perfluorocyclobutane groups at lower temperatures than are needed with such groups as perfluoroalkyl groups and are more stable than monomers where the perfluorovinyl group is attached directly to R. Monomers having such linking structures are also relatively easily prepared.

R is suitably any inert aromatic hydrocarbyl group, preferably a molecular structure which facilitates formation of perfluorocyclobutane rings and/or polyfunctional crosslinking and/or imparts desirable physical properties to polymers or oligomers prepared from the monomers. Additionally, R optionally contains groups or has substituents which are inert, that is which do not undesirably interfere with the formation of perfluorocyclobutane rings from perfluorovinyl groups. Inert substituents include ether, carbonyl, ester, tertiary amide, carbonate, sulfide, sulfoxide, sulfone, nitrile, alkyl phosphonate, tertiary amine, alkyl phosphate, alkyl silyl, chlorine, bromine, fluorine, alkyl, arylalkyl, alkylaryl, cycloalkyl, aromatic, heterocyclic, alkoxyl and aryloxy groups, which inert substituents are suitably in any position, for instance, in a polymer backbone between X's and/or appended to such a backbone. Carbon-containing inert substituents on R preferably contain from 1 to 50, more preferably from 1 to 12 carbon atoms because of the stability and ease of working with monomers of lower molecular weight. R, including inert substituents preferably has a molecular weight (MW) of from 14 to 20,000, more preferably from 75 to 15,000 and most preferably from 75 to 5,000. These ranges include monomeric and oligomeric R groups. In the case of monomers which are other than oligomeric, R preferably has 6 to 50 carbon atoms because molecular weights above this reduce the contribution to properties made by the fluorine-containing substituents. As previously discussed, the nature of R as well as the perfluorocyclobutane content of the polymers can vary broadly according to the type of products desired.

For polymers having good plastic properties such as tensile strength and flexibility, at least one carbon atom of R is in the molecular chain between X's and is part of an aromatic nucleus. Aromatic groups are desirable because of improved physical properties of the polymers and ease of manufacture of the monomers. For both ease of manufacture of the monomer and monomer stability, each X is preferably independently sulfur or oxygen. The aromatic group can be any molecular structure having aromatic character, advantageously having at least one six-membered aromatic ring, suitably having any number of such six-membered rings fused together or connected by bonds or linking structures. R preferably has from 1 to 50 such rings, more preferably from 1 to 10 rings, more preferably containing from 6 to 25 carbon atoms, most preferably R has at least 2 to 4 aromatic rings to impart properties such as hardness and/or stiffness to a polymer. The aromatic fragment is suitably unsubstituted or inertly substituted. Inert substituents on an aromatic R include, for instance, the inert substituents listed for R generally. Exemplary aromatic molecular fragments include, for instance, perchlorophenylene, phenylene, biphenylene, naphthylene, dichlorophenylene, nitrophenylene, p,p'(2,2-diphenylene propane) [$-C_6H_4-C(CH_3)_2-C_6H_4$]; p,p'-,2,2-diphenylene-1,1,1,3,3,3 hexafluoropropane) [$-C_6H_4-C(CF_3)_2-C_6H_4-$], preferably biphenylene; phenylene: 9,9'-diphenylfluorene, oxydiphenylene: thiodiphenylene; 1,1,1-triphenyleneethane; 1,3,5-triphenylenebenzene; 1,3,4-(2-phenylene-2-propyl)benzene; 1,1,1-triphenylenemethane; 1,1,2,2-tetraphenylene-1,2-diphenylethane;

bis(1,1-diphenyleneethyl)benzene; 1-(2-phenylene-2-propyl)-4-(1,1-diphenyleneethyl)benzene; 2,2-diphenylene propane; 2,2'-diphenylene 1,1,1,3,3,3-hexafluoropropane; 1,1-diphenylene-1-phenylethane; naphthalene; and anthracene. Molecular weights of aromatic ring containing polymers are preferably at least about 10,000. Such aromatic groups are preferably present because they generally impart high temperature glass transition properties (Tg) and good mechanical strength (for example, as measured by differential scanning calorimetry (DSC) and tensile/flexural tests) to the polymer.

For the purpose of facilitating backbone crosslinking, more preferably, R is a group which reacts with perfluorovinyl groups residual in a substantially linear polymer to form a crosslinked or branched molecular structure. The reaction of R with the perfluorovinyl groups is suitably initiated by heat, free radicals, wave energy, or any other crosslinking initiating means, but preferably by heat. Most preferably R includes a structure having two double, triple or aromatic bonds (hereafter multiple bonds) separated by a single bond. Such structures are recognized in the arc as latent dienes. Preferably the latent dienes are suitable for reactions of the Dieis-Alder type, more preferably suitable for such reactions with perfluorovinyl groups in the monomers, most preferably suitable for such reactions with perfluorovinyl ether groups under conditions used for crosslinking. The single bond is preferably a carbon to carbon single bond. Each of the multiple bonds is independently suitably a multiple bond between any two atoms, preferably between a carbon atom and any other atom (for example, -C = O, -C = C-, -C≡N), more preferably a carbon to carbon bond. Exemplary of preferred R groups include, for instance, biphenylene, 9,9'-diphenylfluorene, flourene, cyclopentadienylene, furan and anthracene.

Even though Diels-Alder reactions of perfluorovinyl groups are rare (See D. D. Coffman, et al., J. Am.Chem. Soc., 71, 490-496 (1949); E. T. McBee, et al., J. Am. Chem. Soc., 77, 915-917 (1955); J. J. Drysdale, et al., J. Am. Chem. Soc., 80, 3672-3675 (1958)), monomers capable of such reactions are observed to give backbone crosslinked polymers having gel points at temperatures generally lower than similar polymers formed from monomers wherein double bonds are separated by more than one single bond.

Most preferably, at least one aromatic carbon atom of R is bonded directly to X, most preferably aromatic carbon atoms of R are bonded directly to each X because perfluorovinyl groups bonded to X, said X being bonded to aromatic groups are generally more reactive in forming perfluorocyclobutane rings.

At east one X is preferably other than a bond, more preferably neither X is a bond, because attachment of perfluorovinyl groups directly to aromatic R renders the perfluorovinyl groups more thermally and oxidatively unstable than when said groups are attached, for instance to oxygen or sulfur.

Monomers useful in the practice of the present invention are suitably prepared by any method which links molecular structures having perfluorovinyl groups to other molecular structures or which forms perfluorovinyl groups.

Perfluorovinyl groups are formed, for instance by elimination of halogens from terminal dihalotrifluoroethyl groups. Halogens such as bromine or iodine may be eliminated, for instance, using metallic reactants as illustrated by Cohen's synthesis of trifluorostyrene by the reaction of zinc with dichlorotrifluoroethylbenzene in absolute ethanol (J. Am. Chem. Soc., 71, 3439, (1949)). Additionally, pentafluoroethyl (substituted) phenyl ethers can be reacted with certain phosphorus compounds to form perfluorovinyl ethers as reported by Kawaguchi et al. in Japanese Kokai 77 89,603. Structures suitable for elimination of halogens to form perfluorovinyl groups are prepared, for instance, by processes such as those taught in Rico et at. U S-A-4,377,711 and Carl et al. in U S-A-4,423,249. Additionally perfluorovinyl groups are formed by decarboxylation of perfluorocarboxylic acids with concomitant loss of hyorogen fluoride, as taught by R.N. Griffin and M.I. Bro, J. Org. Chem., 25, 1068 (1960), and also by T.S. Reid, G.H. Smith, and W.H. Pearlson, in U S-A-2,746,997. Electrochemical elimination of halogens from certain substituted alkyl-1,2-dihalo-1,2,2-trifluoroethyl ethers according to the procedures taught in European Patent document EP 293,856 is also useful for forming perfluorovinyl compounds.

Tetrafluoroethylene and chlorotrifluoroethylene are reacted with suitable compounds, for instance, by procedures taught by Prober in J. Amer. Chem. Soc., 75, 968 (1953); by Plumer et at. in U.S. Office Saline Water, Res. Develop. Prog. Rep. #481, 1969; by Dixon in J. Org. Chem., 21, 400 (1956); and by Wall et at. in U S-A-3,277,068.

Linking of molecular structures containing perfluorovinyl groups to other molecular structures is illustrated by reaction of (trifluorovinyl)trimethyltin aryl iodides in the presence of palladium complexes, as taught by R.S. Sorokina, et at. in Zh. Org. Khim., 18, 2458, (1982); by the reaction of trifluorovinyl zinc reagents with certain substituted phenyl iodides as taught by R.S. Sorokina et al. in Izv. Akad. Nauk SSSR, Ser. Khim., 1647, (1985); and Heinze and Burton in J. Fluorine Chem., 31, 115, (1986), and J. Org. Chem., 53, 2714, (1988).

Preferred monomers are preferably prepared by the process taught in US-A-5,023,380.

An exemplary method of preparing a tris-perfluorovinyl ether (exemplary of monomers having three perfluorovinyl groups) is illustrated by a process having the following steps:

(A) A trihydroxy compound such as 1,1,1-tris(4-hydroxyphenyl)ethane is converted to its sodium or potassium salt in a solvent such as a methanol/water mixture. The methanol or another solvent for the trihydroxy compound is used when, as in the case of 1,1,1-tris(4-hydroxyphenyl)ethane, the compound is not water soluble. Sufficient alcohol is used to keep the trihydroxy compound in solution; in the case of 1,1,1-tris(4-hydroxyphenyl)ethane from about 20 to about 50 volume percent based on total volume of alcohol and water is convenient. Salt formation occurs conveniently at from about 0 °C to about 60 °C at atmospheric pressure, preferably under a nitrogen atmosphere to avoid oxidation.

(B) The methanol is removed under reduced pressure at any convenient temperature and pressure with replacement of water as it is lost.

(C) The salt is dried and powdered for example, in a drum dryer.

(D) The salt is slurried in a polar, aprotic solvent suitable for achieving reaction such as DMSO (dimethyl sulfoxide), ether diglyme, DMF (dimethyl formamide) HMPA (hexamethylphosphoramide) diglyme, tetraglyme or glyme and an aprotic azeotropic medium such as toluene or chlorobenzene in a solvent to azeotrope medium ratio of from 10 to 1 to 1.5 to 1 and dried by the azeotropic removal of water.

(E) About half of the toluene is removed, for example, by distillation and the mixture is cooled below about 50°C, preferably below about 20°C.

(F) A dihalotetrafluoroethane such as 1,2-Dibromotetrafluoroethane is added to form a mixture as the reaction temperature is controlled at a temperature suitable for the reaction to occur substantially without the side reaction of ring bromination halogenation; in the case of 1,1,1-tris(4-hydroxyphenyl)ethane preferably below about 20°C initially. The mixture is stirred at 18°C to 25°C for 36 hours.

(G) The mixture is poured into about an equal volume of cold water, conveniently from about 0.5 to about 3 times the volume of the solution, and the product falls out as the lower layer. There is preferably sufficient cooling to offset the heat generated by admixing DMSO (or other solvent) and water.

(H) The product, a tris-bromide, is distilled, for example, at 190°C to 195°C 6.67 Pa (0.05 mm Hg). When tris-bromides are heat stable as observed in the case of 1,1,1-tris(4-(2-bromotrifluoroethoxy)-phenyl)ethane, the degree of vacuum is selected to give a convenient boiling point. Such selection is within the skill in the art.

(I) The tris-bromide is used directly or, if desired, in cases where the tris-boromide is a solid it may be dissolved for ease of addition in a polar, aprotic solvent such as diglyme, tetraglyme or glyme and added to a hot (for example 120°C) mixture of the same solvent and granular zinc to form the tris-perfluorovinyl ether (TVE). Alternatively, the tris-bromide can be added to a hot (for example 120°C) mixture of, for example, diglyme and granular zinc as a melt without dilution if heated above its melting point, for example to about 120°C In the case of 1,1,1-tris(4-(2-bromotrifluoroethoxy)phenyl)ethane. Temperatures above about 125°C are preferably avoided to avoid dimerization of perfluorovinyl groups.

(J) The TVE is isolated by removing the zinc salts for example by centrifugation, evaporating the diglyme under reduced pressure, diluting the TVE with a, preferably low boiling, solvent such as hexane, and flushing the solution through a pad of neutral alumina. Alternatively, the zinc salts are removed by filtration and the TVE distilled under vacuum, for example in two stages, the first to remove solvent and the second to purify the TVE. Preferably, temperatures above about 110°C are avoided to avoid dimerization of perfluorovinyl groups. Especially when a very pure product is desired, these methods of purification are suitably combined.

K) The hexane, if used, is removed from the TVE by evaporation under reduced pressure.

Alternatively, the TVE is isolated by removing the zinc salts by filtration, evaporating the diglyme under reduced pressure, diluting the TVE with hexane and purifying by countercurrent extraction with polar organic materials such as acetonitrile or DMSO. The pure TVE in hexane is then flushed through a pad of alumina and isolated by concentration under reduced pressure.

Polymers produced from the preferred monomers preferably have a formula represented by the following Formula II:

Formula II

$$-[X-R-[X-Q]_m]_n-$$

wherein R, X, and m, are defined above, Q is a perfluorocyclobutane group; and n is an integer representing the number of repeating units, which is preferably from 2 to 100,000. More preferably from 2 to 10,000, most preferably from 3 to 5,000. More preferably m is one or two. Formula II is generalized; when m is greater than one, some of the -X- Q structures represent branching and/or crosslinking.

The monomers are heated to a temperature and for a time sufficient to form perfluorocyclobutane rings. Temperatures suitable for forming perfluorocyclobutane rings differ with the structure of the monomer. In general, temperatures above about 40 °C are suitable for formation of perfluorocyclobutane rings, preferably the temperature is above about 50 °C. more preferably above about 100 °C, because these temperatures result in formation of the rings at successively faster rates. Temperatures above about 450 °C are preferably avoided because perfluorocyclobutane groups are generally thermally unstable above such temperatures. More preferably a temperature of from 105 °C to 350 °C, most preferably from 105 °C to 250 °C is used to produce the perfluorocyclobutane rings at a convenient rate. Within that range, a temperature of from 100 °C to 230 °C is generally most preferred for cyclization of perfluorovinyl aromatic ethers or sulfides, while a temperature of from 50 °C to 80 °C is needed to form perfluorocyclobutane groups when the perfluorovinyl group is attached directly to an aromatic ring.

Preferably, especially when the perfluorovinyl compounds are capable of radical initiated addition polymerization, conditions conducive to free radical polymerization, for example, presence of oxygen, ozone, peroxygen compounds and other free radical generating compounds, are avoided so that the perfluorovinyl groups will dimerize into perfluorocyclobutane groups rather than undergoing addition polymerization. Compounds known in the art for stabilization against free radical polymerization are alternatively used. Similarly, especially when the perfluorovinyl groups are capable of addition polymerization in the presence of anions or cations, compounds which supply such anions or cations are avoided. For instance, fluoride ions (for example, from carbonyl fluorides) chloride, hydroxide and phenoxide are preferably avoided. To avoid such compounds as carbonyl fluorides, oxidative conditions such as presence of oxygen, hypochlorite, dichromate and permanganate are preferably avoided because perfluorovinyl groups are known to oxidize to form carbonyl fluorides. Perfluorovinyl compounds such as perfluorovinyl ethers, thioethers, sulfones and sulfoxides are relatively stable with regard to addition polymerization and oxidation; and, therefore, such precautions are generally unnecessary when such perfluorovinyl compounds are used.

Monomers or admixtures thereof are suitably neat or, optionally, in admixture with other materials such as in solution, in emulsion, in dispersions or in any other form in which monomer molecules can be contacted with one another to form a polymer. Liquid admixtures are advantageous for maintaining contact between monomer molecules such that higher molecular weight polymers are formed. This is particularly useful when linear thermoplastic polymers are the products. Neat polymerization is preferred when the monomers or prepolymers are formed in the final desired shape of the polymer article before final thermal treatment. This is especially true when monomers having more than two perfluorovinyl groups are used in whole or in part to formed crosslinked, thermoset materials. Neat polymerizations or oligomerizations are also generally preferred to form relatively low molecular weight fluid products.

Suitable solvents are those which are inert to the conditions encountered in the polymerization reaction and include, for example, xylene, mesitylene and perfluorotetradecahydrophenanthrene (MULTIFLUOR™ APF 215 commercially available from Air Products Corp.). At atmospheric pressure, preferred solvents are those which attain temperatures of 170 °C to 250 °C such as dichlorobenzene, trichlorobenzene, diphenyl oxide and perfluorotetradecahydrophenanthrene. Although solvents such as 1,2-dichlorobenzene and 1,2,4-trichlorobenzene give less satisfactory results such as discoloration of the finished polymer, they are suitably used when their disadvantages are tolerable in a final product. When a solvent is used the concentration of monomers in solvent is advantageously from 0.1 to 99.9 weight percent preferably, from 10 to 90 percent by weight monomer.

Polymerization or dimerization suitably takes place at any pressure. Pressures are generally chosen such that the monomers and any solvents and/or dispersing media remain liquid at the temperatures used for polymerization. When the monomers or other materials evaporate at temperatures used, then it is generally preferable to maintain a pressure at least sufficient to maintain the materials liquid. Additionally, the pressure for polymerization is any pressure above the pressure at which a monomer boils (or its vapor pressure is equal to that used in the polymerization) at the temperature used for the polymerization preferably from 0.01 mmHg (1.33 Pa) to 20,0000 psig (about 1,000,000 mmHg ($1.3 \times 10^5$ kPa)).

Monomers having three or more dimerizable perfluorovinyl groups (hereinafter referred to as polyfunctional) are especially useful to form polymers having relatively high Tg believed to be due to polyfunctional crosslinking. From 0 to 100 percent by weight of such monomers are suitably used, preferably sufficient of the monomers having at least three perfluorovinyl groups to measurably increase the Tg of the polymer

over that of a polymer of monomers having corresponding structures but with only two perfluorovinyl groups, preferably at least about 0.05 mole percent, more preferably from 0.1 to 100 mole, most preferably at least 0.5 mole percent percent of such monomers is used. While use of lower proportions of polyfunctional monomer(s) produces, generally thermoplastic, polymers having crosslinking and corresponding properties of toughness and solvent resistance, use of sufficient polyfunctional monomers to form thermosetting polymers is useful to produce crosslinked polymers having higher Tg. The relative proportions of polyfunctional monomer which produce such polymers varies with the structure of the monomers. However, from 0.05 to 75 mole percent polyfunctional monomers used with monomers having 2 perfluorovinyl groups is sufficient to result in sufficient crosslinking in a thermoplastic polymer to reduce its solubility in a solvent. Thermoset polymers however are advantageously formed from monomer mixtures having from 75 to 100 mole percent, preferably from 85 to 100 mole percent of the polyfunctional monomers. For instance, a polymer of 1,1,1-tris(4-trifluoroethenyloxyphenyl)ethane which is 97 weight percent pure with the major impurity being 1,1-bis(4-trifluoroethenyloxyphenyl)-1-(4(1,1,2,2-tetrafluoroethoxy)phenyl)ethane is polymerized at 180°C to 240°C for 30 minutes and produces a colorless, transparent polymer having a Tg of 286°C.

While monomers of the invention are conveniently polymerized in a single stage polymerization, use of more than one stage is often advantageous particularly for polyfunctional monomers. A first stage can advantageously be used to react some of the perfluorovinyl groups to reduce the exotherm frequently observed when large quantities of monomers, particularly of monomers having at least three perfluorovinyl groups are polymerized in a single stage. A first stage is also useful to produce a monomer (or monomer mixture or solution) having increased viscosity and lower volatility than those of the initial monomer. A liquid or a solid of relatively lower melting point than that of a completely cured polymer is suitably formed. Such a first stage of polymerization is conveniently carried out at temperatures of from 50°C to 400°C, preferably from 105°C to 250°C, more preferably from 120°C to 170°C. At least one later stage follows the first stage and is preferably carried out at a higher temperature than the first to allow the polymerization to proceed toward completion. Such later stage(s) are conveniently carried out at temperatures from that sufficient to result in additional polymerization up to the decomposition temperature of a resulting polymer, preferably from 100°C to 450°C, preferably from 120°C to 400°C, more preferably from 200°C to 375°C. Those skilled in the art will recognize that the first and later stages can represent more than one stage or can be carried out using two or more temperatures and that a series of stages or a continuum of temperatures are suitably used. In addition to these stages a postcure at relative high temperature such as from 200°C to 450°C is optionally used. The postcure is suitably for any duration sufficient to change physical properties and insufficint to decompose the polymer, preferably from 1 minute to 1 week, more preferably at high temperatures such as from 240°C to 450°C for duration of from 1 minute to 24 hours. Stages of polymerization are conveniently run under conditions previously described for polymerization of the monomers. When a solvent is used in an early stage, and it is desirable to avoid bubbles that may occur as a solvent is driven off, advantageously the solvent is removed before or during a later stage.

In a preferred embodiment of the present invention, conditions suitable for all or part of the dimerization can occur in a polymer shaping apparatus, for instance, an extruder, injection mold or compression mold. This embodiment of the present invention is particularly useful when materials containing perfluorovinyl groups, for instance, monomers, oligomers or polymers, have a viscosity suitable for introducing into the polymer shaping apparatus and the material resulting from the formation of perfluorocyclobutane groups in the apparatus has a higher viscosity or is solid, which is less suitable for introduction into the apparatus. More specifically, a perfluorovinyl containing oligomer or relatively lower molecular weight polymer, including the result of partial dimerization of perfluorovinyl groups is introduced into a shaping apparatus wherein it is heated sufficiently for formation of sufficient perfluorocyclobutane rings to form a solid polymer. This technique is particularly useful when applied to oligomers of materials that would be difficult to fabricate as high molecular weight polymers, but where the properties of the higher molecular weight polymers are desired.

One such preferred embodiment of the present invention suitable for, but not limited to, formation of perfluorocyclobutane rings in a shaping apparatus involves forming trifluorovinyl terminated relatively low molecular weight (for example, from 300 to 30,000, preferably from 1,000 to 20,000, more preferably from 1,000 to 5,000) oligomers or polymers, that have a low viscosity relative to their higher molecular weight counterparts. Suitably low molecular weight polymers of the present invention include, for example, addition polymers (including addition polymers of perfluorovinyl compounds) or condensation polymers such as polyethers, poly(carboxylic acid derivatives) including polyesters, polyurethanes, epoxy resins, polysulfones, polycarbonates and polyamide-polyimides; preferably polycarbonates, polyesters, polyamides and polyimides, more preferably polyimides, liquid crystal polymers, especially polyesters, aromatic polyesters,

aromatic polyamides and aromatic polycarbonates which are frequently intractable or have high melting points and poor melt flow characteristics above temperatures commonly used in shaping or molding polymers, when advanced to high molecular weights such as molecular weights greater than about 10,000. These materials are advantageously prepared from the oligomers or polymers by forming the trifluorovinyl group directly onto the terminal positions using chemistry such as taught in the preceding paragraphs or, more conveniently, by reacting a compound containing a trifluorovinyl group and a second functional group reactive with the terminal functionality of the oligomers or polymers. Examples of suitable second functional groups on the compound containing the trifluorovinyl group are carboxylic acid groups and their derivatives such as salts, acid halides, or esters; amines, either primary or secondary; hydroxyl; chloroformate or any number of other nucleophilic or electrophilic groups. Suitable terminal groups of the oligomers or polymers are the same as described for the trifluorovinyl containing compound. When one of the reactants has a given reactive group, the other has a functional group of opposite reactivity, for example nucleophilic with electrophilic. Preferably the perfluorovinyl group is incorporated as a perfluorovinyl ether, more preferably a perfluorovinyl aromatic ether, most preferably as the perfluorovinyl ether of an aromatic ester, as for example $CF_2 = CF\text{-}O\text{-}Ar\text{-}CO\text{-}O$-oligomer where Ar is an aromatic group. An example of the latter method of preparing the monomer and subsequently the polymer of the present embodiment of the present invention is the reaction of polycarbonate oligomer or other oligomer having terminal phenolic groups with 4-trifluorovinyloxybenzoyl chloride. The resulting oligomeric monomer is terminated with trifluorovinyl groups connected to the oligomer via ester groups formed in the reaction of the phenolic end groups with the acid chloride reactive site of the trifluorovinyl compound. The reaction is conveniently conducted by methods of forming esters from phenolics and acid chlorides. Oligomers, thus capped, are then thermally polymerized to a higher molecular weight polymer wherein the oligomer fragments are linearly linked by perfluorocyclobutane rings. Polymers, thus formed, retain substantial property similarity to high molecular weight resins of the oligomer structure. Preparation of trifluorovinyl compounds having second functional groups are prepared according to procedures outlined above and taught in US-A-5,037,919 and US-A-5,021,602. The 4-trifluorovinyloxybenzoyl chloride referred to above and related compounds are prepared from phenolic substituted aromatic esters by techniques taught in U S-A-4,423,249, followed by hydrolysis to the acid and then conversion to the corresponding acid chloride. The above technique is equally applicable to polyfunctional compounds other than oligomers of polymers. Thus, a difunctional compound such as dihydroxybiphenyl can be reacted with the above acid chloride to form a bis(trifluorovinyl) monomer.

Another particularly useful embodiment of the present invention is the formation of linear, thermoplastic materials from monomers containing two trifluorovinyl groups. Generally monomers containing two vinyl groups result, by conventional addition polymerization, in highly crosslinked, brittle plastics. The present novel polymerization process is more akin to condensation polymerization in that difunctional monomers result in linear polymers. To obtain properties associated with structural plastics, it is preferred that R in Formula I is aromatic and most preferred that R contains more than one aromatic ring. It is also preferred that each X is oxygen or sulfur (optionally as sulfone or sulfoxide) and most preferably oxygen. The resulting aromatic perfluorocyclobutyl ether backbone in the thus formed polymers have exceptional physical properties combined with excellent melt processability on conventional thermoplastic fabrication equipment. A polymer exemplary of these characteristics is represented by Formula II wherein R is biphenyl, m is 1, and each X is oxygen. The monomer is conveniently prepared from 4,4'-dihydroxybiphenyl and dibromotetrafluoroethane by modification of chemistry described in U S-A-4,423,249 or by the process disclosed in U S-A-5,023,380. The monomer is easily polymerized at a convenient rate by heating in bulk or solution at 150°C to 200°C. Typical properties of a polymer prepared in perfluorotetradecahydrophenanthrene solvent are a glass transition temperature of about 170°C, tensile strength (as measured by the procedure of ASTM D-882-83) of from $34.5 \times 10^3$ to $41.4 \times 10^3$ kPa (5,000 to 6,000 psi), percent elongation (as measured by the procedure of ASTM D-882-83) of from 10 percent to 20 percent, tensile modulus (as measured by the procedure of ASTM D-882-83) of from 200,000 to 300,000, dielectric constant (as measured by the procedure of ASTM D150-87) of from 2.25 to 2.65, and static dissipation factor (as measured by the procedure of ASTM D150-87) of from 0.0001 to 0.0008.

Polymers of the present invention are suitably solids, fluids or gels, preferably solids or fluids, most preferably solids. The solids preferably maintain plastic characteristics such as tensile strength well above ambient temperatures (for example, above about 25°C) and have glass transition temperatures from well below ambient to well above ambient temperatures. A particularly preferred group of such polymers have glass transition temperatures (Tg) above ambient (25°C), preferably above 60°C and most preferably above 100°C. In general, the polymers having Tg above ambient result from monomers of Formula I wherein R is aromatic, and the polymers having Tg above 60°C when R contains more than one aromatic

ring. A particular desirable property of polymers where R is aromatic and not substituted with polar substituents (for example, nitro, sulfonate, carboxy) is the combination of good physical properties and good electrical properties. Dielectric constants and static dissipation factors (as measured according to the procedures of ASTM D150-87) preferably range from 2.2 to 3.0 and from 0.0001 to 0.005 respectively. Glass transition temperatures increase from about ambient when R is phenyl, to about 170 °C when R is biphenyl, to about 230 °C when R is 9,9-diphenylfluorene, to about 286 °C or higher when R is 1,1,1-triphenylethane.

The linear polymers are advantageously thermoformed as by molding or extruding or are cast from solvents such as ethers and chlorinated solvents, for instance, tetrahydrofuran or dichloromethane. The polymers possess processing advantages over other polymers having similar low dielectric properties such as polytetrafluoroethylene. The advantages include extrudability, suitability for injection molding and solvent casting.

Before backbone crosslinking, substantially linear polymers or oligomers are thermally produced from the preferred monomers. The polymers can be crosslinked by heat including radiant heat, for example infrared light. This crosslinking preferably occurs substantially without crosslinking agents such as monomers having three or more functional groups reactive to form the polymers (trifluorovinyl groups). By substantially without, it is meant that while materials which act as crosslinking agents or catalysts for crosslinking may incidentally be present in very small quantities, preferably less than about 0.01 weight percent of the polymer, more preferably less than about 0.01 weight percent crosslinking agent and less than about 0.001 weight percent catalyst for crosslinking, such materials are not deliberately added. Thermal crosslinking does not require addition of a catalyst such as a free radical initiator.

Thermally backbone crosslinked polymers are prepared from such thermally formed polymers containing perfluorocyclobutane rings by heating the polymers to a temperature sufficient to result in crosslinking, that is for chemical bonds to form between at least some of the polymer molecules. The temperature for such crosslinking is higher than that required for thermal (linear) polymerization, preferably it is at least about 50 °C degrees higher than the temperature required for thermal (linear) polymerization, more preferably from 250 °C to 400 °C, most preferably from 280 °C to 380 °C, even more preferably from 280 °C to 340 °C. These temperatures are suitably maintained for a time sufficient to achieve a preselected degree of crosslinking. Such times are preferably from 1 minute to 10 days, more preferably from 15 minutes to 1 day (24 hours), most preferably from 15 minutes to 8 hours.

A particularly useful aspect of the present invention includes preparing the substantially linear polymers according to processes previously taught and exposing the linear polymer to conditions suitable for backbone crosslinking in a shaping apparatus, for example, an extruder, a mold, or other apparatus suitable for heating for example, for a time and at a temperature sufficient for crosslinking. This allows handling of a polymer which melts at a relatively lower temperature and producing an elastomer or thermosetting polymer having stability at temperatures sufficient to melt the substantially linear polymer. Crosslinking advantageously occurs without the need for a solvent or a high pressure apparatus, and proceeds without the evolution of volatile compounds or by-products.

Crosslinked polymers formed by the process of the invention differ from crosslinked polymers prepared from monomer mixtures containing monomers having more than two perfluorovinyl groups in that they are prepared directly from bifunctional monomers or thermoplastic polymers thereof without addition of a catalyst or a multifunctional crosslinking agent. Also, polymers prepared from a single monomer having more than one crosslinking agent in general have a very high crosslink density and are more rigid and inflexible solids than polymers crosslinked by the process of the present invention.

The following examples are offered to illustrate but not to limit the present invention. In each case, percentages are weight percent unless otherwise indicated. Examples (Ex.) of the present invention are indicated numerically, while comparative samples (C.S.) are not examples of the present invention and are indicated with letters.

All gas chromatography/mass spectrometry (GC/MS) analyses of monomers and intermediates are performed on a Finnigan 1020 GC/MS using a 30 meter RSL-150 fused silica capillary column. All GC/MS analyses of fluid polymer samples are performed on a Finnigan 4500 GC/MS using a 60 meter DB-1 fused silica capillary column, with the GC program run at 290 °C isothermal. Mass to charge (M/e) ratios and percentage (%) of peak height relative to tallest (parent) peak are given. Liquid chroamatography/mass spectrometry (LC/MS) is performed on a Finnigan 4500 mass spectrometer using acetonitrile - water eluent and a moving belt LC/MS interface.

Dynamic Mechanical Spectroscopy (DMS) measurements are performed on a Rheometrics RDS-7700 rheometer in torsional rectangular geometry mode using 60mm x 12mm x 3mm samples at 0.05 percent strain and 1 Hertz (Hz). Differential scanning calorimetry (DSC), thermomechanical analysis (TMA) and

thermogravimetric analysis (TGA) is performed on a Perkin Elmer 7000 thermal analysis system unless otherwise indicated.

Dielectric constant and dissipation factor measurements are conducted according to the procedures of ASTM D150-87. Tensile strength and modulus and percent elongation were measured on an Instron model 1125 according to the procedures of ASTM D-882-83.

Gel Permeation Chromatography (GPC) is performed on a waters 720 GPC instrument using a methylene chloride eluent and a series of Micro-Styragel™ columns of 1,000, 100, 50 and 10 nm (10,000, 1,000, 500 and 100 angstrom) pore sizes. Reported values are standardized against polystyrene.

Granular zinc is activated by washing in 0.1 normal (N) hydrochloric acid (HCl) followed by drying in a vacuum oven at 66.5 Pa (0.5 mmHg) and 140 °C for 10 hours.

Infrared (IR) spectra are measured on a Beckmann Microlab 600 model spectrophotometer. Nuclear Magnetic Resonance (NMR) spectra are measured on a Varian EM360 spectrometer using 19F (fluorine 19) or 1H (hydrogen) mode.

The gel point determination involves a dynamic mechanical spectroscopy technique performed at 10 percent strain in a parallel plate mode using 1 mm gap. Storage and loss moduli are measured at frequencies from 0.1 to 100 radians per second, measuring 10 different frequencies per decade of frequency, every 15 minutes. Measurements are carried out isothermally. The ratio of the loss modulus (G") to the storage modulus (G'), known as the loss tangent, or tan delta (G"/G') is plotted against time for a number of frequencies. The point at which the value of tan delta becomes independent of frequency (as is indicated by a convergence of plots of the log tan delta versus time at various frequencies to a point) is known as the gel point, and represents the time at which the size of the polymer chains in the sample increase to a size which is on the order of the size of the sample. At this point the sample is considered crosslinked on a macromolecular scale, and exhibits properties such as enhanced mechanical strength and insolubility in solvents for the thermoplastic precursor.

EXAMPLE 1: PREPARATION AND BULK POLYMERIZATION OF 4,4'-BIS(TRIFLUOROVINYLOXY)-BIPHENYL

Dimethyl sulfoxide (DMSO) (1800 mL) was placed in a 5-liter 5-necked flask fitted with a mechanical stirrer, a Dean-Stark phase separating trap topped with a nitrogen padded reflux condenser, and a thermocouple attached to a temperature controller. The solvent was stirred and purged of oxygen by blowing in nitrogen through a dip-tube placed below the surface of the liquid while 4,4'-dihydroxybiphenyl (454g, 2.44 mole) was added to the flask.

The system was stirred and purged for 20 minutes, then potassium hydroxide (85 percent pellets) (322g, 4.88 mole) was added slowly. The stirred mixture was then heated to 120 °C. The temperature was held at 120 °C for 1.5 hours, then the heat was turned off and the mixture was allowed to cool to room temperature. Toluene (600 mL) which had been thoroughly purged with nitrogen was added to the solution and the resulting mixture was heated to reflux (135 °C). Water was azeotropically removed from the reactor through the Dean-Stark trap for a total of 4 days, cooling the reactor once after 24 hours to allow for salt formation to be broken up by opening the flask under a nitrogen sweep and scraping the sides with a spatula. After 4 days the Dean-Stark trap was removed and replaced with a Soxhlet extractor containing anhydrous sodium sulfate. The toluene was then refluxed through the Soxhlet extractor for 7 hours to dry the toluene. After 7 hours, the Soxhlet was replaced with a Dean-Stark trap, and toluene (300 mL ) was removed from the reactor by simple distillation. The reaction mixture was then cooled to 30 °C in an ice water bath and 1,2-dibromo-tetrafluoroethane (1300 g, 5.00 mole) was added slowly dropwise over three hours at a rate that maintained a reactor temperature of 35 °±2 °C. When the addition was complete the reaction temperature was allowed to stabilize (not increasing in temperature when the ice bath was removed) and then a heating mantle was applied to the flask. The reactor was heated to 50 °C for 8 hours, then allowed to cool to room temperature with constant stirring. The crude reaction mixture was filtered to remove the potassium bromide salts, and the precipitate is washed with acetone. The filtrates were combined and thoroughly evaporated to remove acetone, DMSO and residual toluene. The solid residue was subjected to a 2-liter Kugelrohr bulb-to-bulb distillation to provide the crude product. This material was dissolved in 750 mL of methylene chloride and was washed first with mild aqueous potassium bicarbonate (500 mL, approximately. 0.2 M), then with mild aqueous hydrochloric acid (HCl) (500 mL, approximately 0.05 M), then twice with distilled water (500 mL each). After complete phase separation the product layer was removed and evaporated, and the residue was fractionally distilled (138 °C to 148 °C, 0.35 mmHg (46.5 Pa)) to provide 1031.1 g (1.90 mole, 77.9 percent yield) of 4,4'-bis(2-bromotetrafluoroethoxy)biphenyl, melting point 71 °C to 73 °C. The Infrared (IR) spectra of the product has the following peaks ($cm^{-1}$):

1601,1492 (indicating an aromatic double bond); 1199-1107 (indicating carbon-oxygen and carbon fluorine bonds); 842, 788 (indicating aromatic character). The gas chromatograph/mass spectrometer (GC/MS) indicated peaks at the following mass to charge ratios (m/e) = 545 (29.8%); 543 (48.9%); 541 (23.8%); 365 (48.7%); 363 (50.9%); 337 (30.3%); 335 (34.7%); 168 (33.7%); 156 (78.3%); 140 (36.7%); 139 (90.1%); 129 (37.4%); 128 (100.0%); 127 (33.2%); 102 (32.9%); 76 (41.1%); 63 (34.3%), consistent with a product of 4,4'-bis(2⁻bromotetrafluoroethoxy)biphenyl.

Bromine was eliminated from this product by the following procedure:

Into a 1-liter 5-necked flask equipped with a mechanical stirrer, a thermocouple attached to a temperature controller, a powder addition funnel and a reflux condenser, was placed freshly distilled diglyme (200 mL) and fresh zinc powder (36.0g, 0.55 mole).

The mixture was stirred and heated to 130 °C. Powdered 4·4'-bis(2-bromotetrafluoroethoxy)biphenyl (100g, 0.184 mole) was added very slowly via the powder addition funnel over 3.5 hours. The mixture was then stirred mechanically at 115 °C for 1 hour, after which heating was turned off and the mixture was allowed to cool to room temperature. The solution was centrifuged to remove the zinc salts. Then the liquid was decanted, and the zinc salts were washed with acetone and centrifuged again. The liquid portions were combined and evaporated thoroughly, and the residue was dissolved in methylene chloride and washed with 0.05 M hydrochloric acid. The methylene chloride solution was evaporated to provide 62.45 g (0.180 mole) of 4·4'-bis(trifluorovinyloxy)biphenyl of 94.5 percent purity in 98 percent yield.

The product was then recrystallized in an ethanol/water mixture to give product of 99.8 percent purity in greater than 70 percent recovery, melting point 44 °C to 46 °C.

The IR spectrum showed peaks at ($cm^{-1}$): 1833 (indicative of a perfluorovinyl group); 1601,1491 (indicative of an aromatic double bond); 1231, 1196-1132 (indicative of carbon-oxygen and carbon-fluorine bonds respectively); 818 (indicative of aromaticity).

The GC/MS spectrum had the following peaks: m/e: 346 (31.3%); 153 (13.8%); 152 (100.0%); 151 (27.0%); 150 (11.7%); 76 (14.9%); 63 (14.9%).

Differential scanning calorimetry (DSC) analysis of the 4,4'-bis(trifluorovinyloxy)biphenyl monomer (20 °C to 360 °C at 20 °C/minute) indicated a sharp endotherm of melting beginning at 45 °C, followed by a broad exotherm beginning at about 170 °C, interpreted as corresponding to the heat of cyclization of the trifluorovinyl groups to form hexafluorocyclobutane rings.

The monomer, 4,4'-bis(trifluorovinyloxy)biphenyl, (15.0g, 0.043 mole) was placed in a nitrogen purged 100 mL round bottom flask and polymerized by heating at 210 °C for 2 hours without stirring. After cooling, a small sample was removed for analysis by differential scanning calorimetry (DSC). The sample shows a small crystalline melt with a peak at 60 °C, followed by a broad exotherm beginning at about 200 °C. The bulk sample was heated again at 235 °C for an additional 3 hours. Again a sample was removed and analyzed by DSC. The analysis indicated a very small crystalline melt with a peak at 60 °C, followed by a low intensity exotherm beginning at about 230 °C. The bulk sample was heated again to 265 °C for 45 minutes. Analysis of this sample indicated no crystalline melt and no exothermic activity up to and including 325 °C, with the emergence of an endothermic glass transition (Tg) at 143 °C.

EXAMPLE 2: POLYMERIZATION OF 4,4'-BIS(TRIFLUOROVINYLOXY)BIPHENYL IN SOLUTION

The monomer, 4,4'-bis(trifluorovinyloxy)biphenyl, (60.0g, 0.173 mole) was placed in a 1-liter 3-necked round bottom flask with 75 mL of perfluorotetra-decahydrophenanthrene (Multifluor™ APF 215 commercially available from Air Products). The flask was fitted with a mechanical stirrer and a nitrogen padded reflux condenser. After purging the flask thoroughly with nitrogen, the mixture was stirred and heated to reflux. Initially, upon heating the melted monomer was not miscible with the solvent, but as the temperature rose the two phases became homogeneous. After stirring at reflux for approximately 45 minutes, a polymer phase separated; and, after stirring at reflux for a total of 3 hours, the phase separated polymer became viscous enough to seize the stirring shaft. The cooled polymer was removed from the flask and evaporated under high vacuum (approximately 66.5 Pa (0.50 mmHg)) at about 220 °C for 3 hours to remove residual solvent. A portion of this polymer was compression molded at 250 °C to provide a light yellow, transparent flexible plastic film. Another portion was dissolved in tetrahydrofuran and placed in an evaporating dish to make a solvent-cast film. After the solvent was evaporated overnight, a light yellow thin film is peeled from the dish. This sample exhibited excellent flexibility and transparency.

An IR spectrograph of the film had the following peaks ($cm^{-1}$): 1601, 1490 (indicating aromatic double bonds); 1302, 1194-1115 (indicating carbon-oxygen and carbon-fluorine bonds), 818 (indicating aromaticity).

DSC analysis of this polymer indicated a Tg transition at 148 °C.

Dynamic mechanical analysis (DMS) gave a Tg value of 170 °C, and gel permeation chromatography (GPC) indicated a weight average molecular weight of 85,000 as standardized against polystyrene.

Dielectric constant and dissipation factor measurements performed on this polymer gave the following results:

| Frequency (kHz) | Dielectric Constant | Dissipation Factor |
|---|---|---|
| 1.0 | 2.58 | 0.0007 |
| 10.0 | 2.57 | 0.0004 |
| 1000.0 | 2.55 | 0.0004 |

Examples 1 and 2 illustrate two types of polymerization of 4,4'-bis(trifluorovinyloxy)biphenyl. It is notable that the properties of each are roughly similar, with slightly more discoloration taking place in the bulk polymerization (according to the procedures of Example 1).

EXAMPLE 3: PREPARATION AND POLYMERIZATION OF 9,9-BIS(4'-[TRIFLUOROVINYLOXY]PHENYL)-FLUORENE

Into a 2 liter 5-necked round bottom flask fitted with a mechanical stirrer, Dean-Stark trap topped with a nitrogen padded reflux condenser and a thermocouple attached to a temperature controller, were placed DMSO (650 mL) and toluene (200 mL). While the stirred solution is purged with nitrogen, 9,9-bis(4'-hydroxyphenyl)fluorene (200.0 g, 0.57 mole) was added to the flask. While purging with nitrogen continued, potassium hydroxide (85 percent pellets, 77.5 g, 1.17 mole) was added all at once, and the mixture was heated to 100 °C with constant stirring. After two hours, the temperature was increased until the solution begans to reflux (130 °C). Water was removed by azeotropic distillation for 24 hours. The Dean-Stark trap was replaced by a Soxhlet extractor containing anhydrous sodium sulfate, and the toluene was refluxed through the Soxhlet for 5 hours. A small amount of toluene (60 mL) was then removed by simple distillation. Then the reactor was cooled to 35 °C. Addition of 1,2-dibromotetrafluoroethane (315 g, 1.21 mole) via dropping addition funnel was then maintained at a rate that keeps the reaction temperature at 35 °C to 38 °C. When the addition was complete, the mixture was heated at 50 °C for 8 hours, then cooled to room temperature with constant stirring. The mixture was filtered, and the precipitate was washed twice with acetone. The filtrates are combined and evaporated thoroughly. The residue from the evaporation was washed with water to remove residual potassium bromide (KBr). After the residue was air dried for 24 hours, it was extracted with hexane in a Soxhlet extractor. Evaporation of the hexane, followed by column chromatography of the residue (on neutral alumina, using hexane eluent) provided a product, 9,9-bis(4'-[2''bromotetrafluoroethoxy]phenyl)-fluorene (331.4 g, 0.468 mole, 82 percent yield), melting point 157 °C to 158 °C.

The LC/MS spectrum had peaks at: m/e:

710 (53.0%); 709 (34.0%); 708 (100.0%); 707 (23.3%); 706 (49.8%); 513 (28.4%); 511 (28.5%); 438 (12.8%); 437 (52.4%); 436 (14.7%); 435 (55.8%); 355 (15.7%); 290 (33.9)%; 289 (19.5%); 239 (35.9%); 228 (36.2%); 227 (38.9%); 226 (47.3%); 202 (27.7%); 157 (47.2%); 131 (27.6%); 129 (23.1%).

The product from the above reaction (18.85g, 0.027 mole) was combined with freshly activated granular zinc (5.00g, 0.076 mole) in glyme and heated at reflux overnight. After cooling, the reaction mixture was decanted and centrifuged to remove suspended zinc salts. The solvent was removed by vacuum evaporation, and the residue was purified by column chromatography on neutral alumina using hexane as an eluent to provide as product 9,9-bis(4'-trifluorovinyloxyphenyl)fluorene (5.55g, 0.011 mole, 40 percent yield), melting point 115 °C to 116 °C.

The LC/MS spectrum had peaks at: m/e:

511 (29.3%); 510 (91.9%); 337 (37.2%); 316 (16.1%); 315 (19.7%); 313 (12.8%); 241 (15.5%); 240 (52.8%); 239 (100.0%); 237 (15.6%); 207 (14.1%); 158 (28.7%); 157 (53.1%); 155 (14.4%); 150 (28.8%); 145 (18.3%); 144 (16.5%); 120 (15.1%).

Into a 50 mL round bottom flask fitted with a nitrogen padded reflux condenser, mechanical stirrer and a thermocouple attached to a temperature controller were placed 9,9-bis(4'-trifluorovinyloxyphenyl)fluorene (3.0g, 0.0059 mole) and diphenyloxide (5.0 mL). The mixture was stirred and heated to reflux (255 °C) for 22 hours. The diphenyloxide (DPO) solvent was evaporated under high vacuum on a 100 milliliter Kugelrohr bulb to bulb apparatus (0.03 mm, 165 °C) to provide the polymer product, which was dissolved in methylene chloride and cast into a thin film.

Gel permeation chromatography analysis of the polymer indicated a weight average molecular weight of 135,000 as standardized against polystyrene.

DSC analysis indicated a Tg transition at 224 ° C.

Example 3 illustrates preparation and polymerization of of 9,9-bis(4,4'-trifluorovinyloxyphenyl)fluorene. It is notable that the resulting polymer, which is polymerized in DPO, attains a high molecular weight and forms a solvent cast film with good physical properties such as flexibility. The polymer is soluble in acetone, dichloromethane and tetrahydrofuran.

EXAMPLES 4-9: PREPARATION AND POLYMERIZATION OF A VARIETY OF PERFLUOROCYCLOBUTANE RING-CONTAINING POLYMERS

The procedure outlined in Example 3 was repeated for each of the indicated starting materials, except for the changes indicated in Table I and adjustments in amounts to maintain the stoichiometry of Example 3, to produce the indicated monomers of the structure:

$CF_2 = CF\text{-}O\text{-}R\text{-}O\text{-}CF = CF_2$

wherein R is given in Table I

Table I: Preparation of Monomers

| EX. NO. | Starting Material | R | Changes in Procedure |
|---|---|---|---|
| 4 | Resorcinol | 1,3-Phenylene | Tetraglyme was used in second step. Product was distilled directly from reaction mixture under vacuum. *3-(1',1',2',2'-tetrafluoroethoxy)-trifluorovinyloxy benzene and 1,3-bisphenol(1',1',2',2'-tetrafluoro-ethoxy)trifluorovinyloxybenzene were isolated as by products and identified by GC/MS spectra consistent with those compounds. |
| 5 | 4,4'-dihydroxy-biphenyl | 4,4'-Biphenyl | See Example 1 |
| 6 | 4,4'-thiodiphenol | 4,4'-Thiodiphenyl | Tetraglyme was used in second step, removed by diluting with methylene chloride and washing with water. |
| 7 | Bisphenol A | Isopropyl-2,2-diphenylene | |
| 8 | Hexafluorobisphenol A (bisphenol AF) | Hexafluoroisopropyl-2,2-diphenylene | |
| 9 | 9,9-bis(4'-hydroxyphenyl)fluorene | 9,9-bis(4'-phenylene)fluorene | See Example 3 |

The data in Table I shows that a variety of perfluorovinyl monomers are prepared by processes within the scope of the present invention.

The procedure outlined in Example 2 was repeated for each of the monomers in Table I, except for the changes in procedure indicated in Table II to produce polymers from the indicated monomers. The properties of these polymers are given in Table II.

Table II

| Properties of Thermoplastic Polymers | | | | | |
|---|---|---|---|---|---|
| Ex. No. | R | Tg (°C) | Dielectric Constant 10 kHz | Dissipation Factor 10 kHz | Weight Average Molecular Weight |
| 4 | 1,3-Phenylene | 32 | 2.41 | -- | 41,400 |
| 5 | 4,4'-Biphenyl | 170 | 2.57 | 0.0004 | 85,000 |
| 6 | 4,4'-Thiodiphenyl | 78 | 2.62 | 0.0005 | 42,500 |
| 7 | Isopropyl-2,2-diphenylene | 98 | -- | -- | 50,700 |
| 8 | Hexafluoroisopropyl-2,2-diphenylene | 125 | -- | -- | 23,500 |
| 9 | 9,9-bis(4'-phenylene)fluorene (prepared in diphenyloxide) | 224 | -- | -- | 135,000 |

The data in Table II shows that a variety of perfluorocyclobutane ring-containing polymers are prepared by processes within the scope of the present invention.

EXAMPLE 10: PREPARATION OF 1,1,1-TRIS(4'-TRIFLUOROVINYLOXYPHENYL)ETHANE AND BULK POLYMERIZATION THEREOF WITH 4,4'BIS(TRIFLUOROVINYLOXY) BIPHENYL

A 1 liter 5-necked round bottom flask was fitted with a mechanical stirrer, a Dean Stark trap topped with a nitrogen padded reflux condenser, and a thermocouple attached to a temperature controller. A mixture of DMSO (450 mL), toluene (150 mL), and 1,1,1-tris(4'-hydroxyphenyl)ethane (55.1 g, 0.18 mole) was added to the flask under nitrogen purge. After stirring for 15 minutes under a vigorous nitrogen purge, potassium hydroxide (85 percent pellets, 80.0g, 1.2 mole) was slowly added to the reaction flask. The mixture was then stirred at reflux for 48 hours with azeotropic removal of water. The resulting suspension was cooled to 35°C in an ice bath and 1,2-dibromotetrafluoroethane (155 g, 0.60 mole) was added at a rate that maintained a temperature of 30°C to 35°C. When the addition was complete, the mixture was heated to 50°C with continuous stirring for 3 hours. After filtration, the solvents were removed by heating under vacuum on a rotary evaporator. The brown residue was purified by column chromatography on neutral alumina using hexane as eluent to provide as product 1,1,1-tris(4'-[2''-bromotetrafluoroethoxy]phenyl)ethane (18.3 g, 0.022 mole, 12 percent yield).

Identity of the product was confirmed by a GC/MS spectrum, the following peaks: m/e: parent ions m/e 840-842-844-846 (ratio 1:3:3:1) too heavy to detect. Structure determined from fragmentation: 573 (32.3%); 571 (58.3%); 569 (31.5%) [indicating parent $-PhOCF_2CF_2Br$]). 299 (58.1%); 297 (52.7%): 279 (32.3%); 228 (43.5%); 227 (31.5%); 226 (36.0%); 215 (59.5%); 181 (82.1%); 179 (100.0%): 165 (50.3%); 152 (43.7%); 131 (47.1%); 129 (50.4%); 100 (38.8%).

Into a 500 mL 5-necked flask fitted with a mechanical stirrer, a reflux condenser, and a thermocouple attached to a temperature controller was placed freshly activated granular zinc (4.3 g, 0.066 mole) and 25 mL dry diglyme. This mixture was stirred and heated to 110°C under nitrogen while the product from the above reaction (18.0 g, 0.021 mole) was dissolved in 21 mL diglyme and added dropwise. The resulting mixture was stirred at 115°C for 3 hours, then cooled and filtered. The filtrate was evaporated at 60°C under vacuum to remove the diglyme, and the residue was purified by column chromatography on neutral alumina using hexane as eluent to provide the product 1,1,1-tris(4'-trifluorovinyloxyphenyl)ethane (9.98 g, 0.018 mole, 8 percent yield).

The GC/MS spectrum had the following peaks:
m/e: 546 (3.2%); 531 (44.0%); 434 (17.9%); 373 (24.4%); 276 (16.9%); 240 (28.1%); 239 (73.9%); 199 (19.3%); 178 (100.0%); 177 (17.8%); 176 (25.4%); 163 (17.3%); 152 (31.9%); 151 (17.8%); 127 (20.3%); 126 (28.7%); 120 (39.1%); 119 (70.3%); 118 (25.6%); 113 (27.3%); 107 (18.8%); 102 (31.7%); 77 (15.9%); 76 (29.5%).

This example illustrates preparation of a trifunctional monomer, 1,1,1-tris(4'-trifluorovinyloxyphenyl)-ethane. This monomer is useful alone or mixed with a bifunctional monomer to produce a crosslinked perfluorocyclobutane polymer.

A mixture of 4,4'-bis(trifluorovinyloxy)-biphenyl (as prepared in Example 1) (4.50 g, 0.013 mole) and the 1,1,1-tris(4'-trifluorovinyloxyphenyl)ethane (0.79 g, 0.0014 mole) were combined in a 100 mL single-necked round bottomed flask topped with a nitrogen padded reflux condenser. The flask was purged thoroughly with nitrogen, and the mixture was heated without stirring. After reaching a temperature of 200 °C, the mixture set into a rigid plastic within 15 minutes. This material was then cured an additional 40 minutes at 220 °C; then the heat was removed. The resulting plastic was rigid, inflexible and did not dissolve in tetrahydrofuran (THF) or methylene chloride, but swelled into a gel in these solvents.

DSC analysis (25 °C to 350 °C, 20 °C/minute) of this polymer sample showed a slight endothermic event at 125 °C followed by a broad exotherm beginning at about 210 °C, indicative of an incompletely cured polymer. After this sample was cured during the first DSC scan, a second scan was run which clearly indicates a Tg transition at 151 °C and no subsequent exothermic activity at higher temperatures.

Example 10 illustrates preparation of 1,1,1-tris(4'-trifluorovinyloxyphenyl)ethane and copolymerization thereof 4,4'-bis(trifluorovinyloxy)-biphenyl therewith. The resulting polymer was stiff and brittle, as well as insoluble, compared to the thermoplastic of Example 2, prepared from 4,4'-bis(trifluorovinyloxy)biphenyl alone, which was flexible and soluble in THF and methylene chloride.

EXAMPLE 11: BULK POLYMERIZATION OF 4,4'-BIS(TRIFLUOROVINYLOXY)BIPHENYL WITH SUBSEQUENT ADDITION OF 1,1,1-TRIS(4'-TRIFLUOROVINYLOXYPHENYL)ETHANE

Monomer 4,4'-bis(trifluorovinyloxy)biphenyl (16.2 g, 0.047 mole) was placed in a 500 mL round bottom flask along with a magnetic stirring bar. A nitrogen padded reflux condenser was placed on the flask, and the monomer was heated at 200 ° to 205 °C with stirring for 20 minutes, to form a low molecular weight polymer resembling a thick fluid at 200 °C. The fluid was allowed to cool to room temperature where it sets into a brittle glass. The glass was dissolved in methylene chloride and 1,1,1-tris(4'-trifluorovinyloxyphenyl)-ethane (0.51 g, 0.00094 mole) was added to the solution. The methylene chloride was evaporated and the residue was dried and devolatilized on a Kugelrohr bulb to bulb apparatus at 120 °C to 140 °C and 26.6 Pa (0.20 mmHg) pressure. While still hot, the fluid mixture was poured into a mold and cured on a hot press at 250 °C and 1.38 x $10^5$ kPa (20,000 psi)for one hour. The mold was removed from the press and cooled. A coupon was removed from the mold. The coupon was a strong and flexible plastic, and did not dissolve in THF but swelled into a gel therein.

DSC analysis of this crosslinked polymer sample indicated a Tg value of 149 °C, with no subsequent thermal activity up to and including 350 °C.

Example 11 illustrates polymerization of 4,4'-bis(trifluorovinyloxy)biphenyl with subsequent addition of 1,1,1-tris(4'-trifluorovinyloxyphenyl)-ethane. It is notable that crosslinked polymers are prepared either by copolymerizing difunctional and multi-functional monomers, as in Example 10, or by combining a multifunctional monomer with a low molecular weight polymer containing trifluorovinyl end groups as in Example 11.

EXAMPLE 12: PREPARATION OF A FLUID POLYMER OF 1,3-BIS(TRIFLUOROVINYLOXY)BENZENE AND 3-TRIFLUOROVINYLOXY-1',1',1'-TRIFLUOROTOLUENE

To synthesize m-trifluorovinyloxy-1',1',1'-trifluorotoluene, DMSO (400 mL), toluene (140 mL), and 3-trifluoromethylphenol (81.0 g, 0.50 mole) were placed in a 1 liter 3-necked flask equipped with a mechanical stirrer, a Dean-Stark trap and a thermocouple attached to a temperature controller. The stirred solution was purged of oxygen by placing a dip tube below the surface of the solution and allowing nitrogen to be blown into the solution for 15 minutes. Potassium hydroxide (85 percent pellets, 33.7g, 0.51 mole) was added to the flask all at once, and a line to supply nitrogen was attached to a reflux condenser which was placed on top of the Dean-Stark trap. The mixture was then heated to 145 °C and water was removed azeotropically. When water ceased to collect in the Dean-Stark trap, the temperature of the reaction was increased to 155 °C and 100 mL of toluene was removed by distillation, leaving a reaction mixture in the flask.

The reaction mixture was cooled to room temperature, and 1,2-dibromotetrafluoroethane (132.0 g, 0.51 mole) was added slowly using a dropping addition funnel. The mixture was heated to 55 °C for 5 hours, then allowed to cool to room temperature. After the suspended solids have settled, the liquid was decanted away from the precipitate and was retained as a mixture of product in DMSO, which was added to a 3 times volumetric excess of water in a separatory funnel and shaken vigorously. The product formed a separate, lower layer at the bottom of the funnel and was removed. This crude product (lower layer) was washed again with 500 mL of water. After drying the washed lower layer over anhydrous magnesium sulfate, the crude product was fractionally distilled. The product, m-(2-bromotetrafluoroethoxy)-1',1',1'-trifluorotoluene (169 °C to 171 °C, 150 mmHg (19950 Pa)) gave the following mass spectral data: m/e: 342 (20.1%); 340

(19.8%); 323 (7.9 %); 321 (7.2 %); 211 (25.6 %); 145 (100.0%).

The product of the above reaction (56.0g, 0.164 mole) was combined with granular zinc (12.0g, 0.18 mole) in dry tetraglyme and stirred at 115 °C for 6 hours to form a reaction mixture. The mixture was cooled to room temperature, and a distillation head was placed on the reaction flask. The product was then distilled directly out of the crude reaction mixture (108 °C to 110 °C, 19950 Pa (150 mmHg) to give 40.5 g of the product, m-trifluorovinyloxy-1',1',1'-trifluorotoluene, which was 78 percent pure by GC analysis, with the remainder of the product being the by-product m-(1,1,2,2,-tetrafluoroethoxy)-1',1',1'-trifluorotoluene.

The product gave the following mass spectral data: m/e: 242 (52.3%); 223 (12.3%); 195 (14.2%); 145 (100%): 125 (18.3%); 95 (30.6%).

A mixture was prepared from 1.25 mL of 1,3-bis(trifluorovinyloxy)benzene (as prepared in Example 4) and 8.75 mL of 3-trifluorovinyloxy-1',1',1'-trifluorotoluene to make a total of 10 mL. This mixture was placed in a 50 mL round bottom flask fitted with a nitrogen padded reflux condenser and was refluxed under nitrogen for 20 hours.

The resulting product was analyzed by GC/MS and found to be a mixture of hexafluorocyclobutane products whose major components were 1,2-bis(3'-trifluoromethyl-phenoxy) hexafluorocyclobutane and 1,3-bis(2'-[3''-trifluoromethylphenoxy]hexafluorocyclobutyl)phenyl ether (having two perfluorocyclobutane rings), with a trace amount of 1,2-bis(3'[2''-{3'''-trifluoromethyl-phenoxy}hexafluorocyclobutyloxy]phenyl) hexafluorocyclobutyl ether (having three perfluorocyclobutane rings). By vacuum distillation two fractions were collected.

The first fraction contained primarily mono-perfluorocyclobutane material consisting of two isomers (cis and trans 1,2-substituted hexafluorocyclobutane) with similar mass spectra (given for one isomer only): m/e: 484 (20.2%); 465 (12.9%); 273 (29.2%); 242 (30.1%); 207 (11.2%); 195 (13.0%); 145 (100.0%).

The second fraction contained predominantly di-perfluorocyclobutane material, consisting mainly of three isomers (cis-cis, cis-trans, and trans-trans) of 1,2-substituted hexafluorocyclobutanes, and small amounts of four isomers of a product containing one 1,2-substituted hexafluorocyclobutane ring and one 1,3-substituted hexafluorocyclobutane ring (cis-1,2 cis-1,3; cis-1,2 trans-1,3; trans-1,2 cis-1,3; and trans-1,2 trans-1,3). All seven products gave roughly the same peaks in their mass spectra in differing intensities. The following mass spectral data was for the first product isomer to elute from the gas chromatography (GC) column, and corresponded to one of the three main isomers of two perfluorocyolobutane rings: m/e: 754 (36.4%); 593 (12.5%); 492 (14.1%); 415 (21.9%); 273 (27.7%); 242 (39.1%); 195 (21.5%); 173 (23.4%); 145 (100.0%); 126 (28.5%); 95 (23.1%); 92 (34.7%); 76 (57.6%); 64 (27.3%).

The second fraction also contained a small amount of material containing three perfluorocyclobutane rings, consisting of six isomers (cis-cis-cis, cis-cis-trans, cis-trans-cis, cis-trans-trans, trans-cis-trans, and trans-trans-trans) of 1,2-substituted hexafluorocyclobutanes. Because of the small amount of this product present in the mixture, the corresponding products containing one or more 1,3-substituted hexafluorccyclobutane rings were not detected. The mass spectra of the six isomers showed roughly the same peaks in slightly differing intensities. The following mass spectral data was from the first product isomer of tri-perfluorocyclobutane material to elute from the GC column: m/e: 1024 (21.6%); 593 (16.3%); 492 (35.5%); 415 (17.6%); 281 (16.2%); 273 (16.4%); 242 (26.0%); 208 (15.9%); 207 (71.9%); 145 (100.0%); 92 (19.7%); 76 (26.8%).

In all cases, the primary products of cyclization were 1,2-substituted hexafluorocyclobutanes, with small amounts (1 to 2 percent) of 1,3-substituted hexafluorocyclobutanes observable by GC/MS, (except for the tri-perfluorocyclobutane material, of which only trace amounts were seen) the two being distinguished by a small peak at m/e = 100, corresponding to a fragment of $CF_2=CF_2$ present in the mass spectra of the 1,2-substituted hexafluorocyclobutanes which was absent in the 1,3-substituted products. Absolute configurations of the different isomers were not assigned.

This example shows that a compound containing one trifluorovinyl group can be combined with a compound containing two trifluorovinyl groups, the mixture then being heated to cause cyclization of the trifluorovinyl groups to provide a fluid containing perfluorocyclobutane groups. Such fluids are of the type useful as low dielectric hydraulic fluids or lubricants.

EXAMPLE 13: PREPARATION OF A FLUID POLYMER OF 1,3-BIS(TRIFLUOROVINYLOXY)BENZENE, 3-(1',1',2',2',TETRAFLUOROETHOXY)-TRIFLUOROVINYLOXYBENZENE AND 1,3-BIS(1',1',2',2'-TETRAFLUOROETHOXY)BENZENE

A mixture (25 mL) consisting of 1,3-bis(trifluorovinyloxy)benzene (as prepared in Example 4) (26 percent), 3-(1',1',2',2',tetrafluoroethoxy)-trifluorovinyloxybenzene (54 percent), 1,3-bis(1',1',2',2'-tetrafluoroethoxy)benzene (as isolated in Example 4) (15 percent),and tetraglyme (5 percent) was placed in

a 100 mL round bottom flask and heated at reflux under nitrogen for 5 hours. The resulting viscous oil was checked by GC and was found to contain unreacted 1,3-bis(1',1',2',2',-tetrafluoroethoxy)benzene and tetraglyme, as well as mixtures of isomers of heavy components. After removal of the light, unreacted components, two fractions are cleanly separated by fractional distillation and each was analyzed by GC/MS.

The first fraction was found to contain primarily 1,2-bis(3'-[1'',1'',2'',2''-tetrafluoroethoxy]-phenoxy)-hexafluorocyclobutane as two isomers (cis and trans substituted hexafluorocyclobutane) followed by small amounts (1 to 2 percent each) of two 1,3-substituted hexafluorocyclobutane products (cis and trans), all having roughly similar mass spectra. The following was the mass spectral data for the first isomer to elute from the chromatography column, and corresponded to one of the 1,2-substituted isomers: m/e: 580 (25.8%); 371 (11.3%); 321 (12.5%); 290 (23.4%); 270 (36.4%); 243 (69.9%); 193 (100.0%); 95 (96.4%); 92 (55.9%); 76 (26.7%); 64 (29.9%); 51 (21.9%).

The second fraction contained 1,3-bis(2'-[3''-{1''',1''',2''',2'''-tetrafluoroethoxy}phenoxy]-hexafluorocyclobutyl)phenyl ether, primarily as three isomers of 1,2-substituted hexafluorocyclobutanes with a small amount of four isomers of the product with one 1,2-substituted and one 1,3-substituted hexafluorocyclobutane ring. The seven isomers all gave roughly the same peaks in their mass spectra in differing intensities. The following mass spectral data was for the first isomer to elute from the GC column, and corresponded to one of the three main isomers of the product: m/e: 850 (24.7%); 540 (24.2%); 371 (41.5%); 321 (12.9%); 301 (16.4%); 290 (33.9%); 270 (74.4%); 243 (63.9%); 207 (24.1%); 193 (86.7%); 173 (14.8%); 95 (100.0%); 92 (63.2%), 76 (71.8%)64 (32.6%); 51 (15.5%).

This example shows that a compound containing one trifluorovinyl group may be combined with a compound containing two trifluorovinyl groups in a solvent, the resulting mixture being heated to cause cyclization of the trifluorovinyl groups. Such fluids are of the type useful as low dielectric hydraulic fluids or lubricants.

EXAMPLE 14: PREPARATION OF METHYL 4-(2-BROMOTETRAFLUOROETHOXY)BENZOATE, ITS CONVERSION TO TO 4-TRIFLUOROETHENYLOXYBENZOIC ACID AND THE BENZOYL CHLORIDE, AND USE OF THE CHLORIDE TO CHAIN EXTEND POLYCARBONATE OLIGOMERS

Methyl 4-hydroxybenzoate (304.3 g, 2 mole) was dissolved in 800 mL of methanol and was converted to the potassium salt by the slow addition of potassium hydroxide (132.02 g, 2 mole, 85 percent purity). The resulting mixture was stirred and cooled as necessary to maintain the temperature below 50 °C. The solvent was then removed by rotary evaporation and the crystalline salt was dried under vacuum overnight at 140 °C.

The dried salt was allowed to cool and transferred to an oven dried 2-liter flask under nitrogen. The flask was fitted with a mechanical stirrer, thermometer, heating mantle, condenser and pressure-equalizing addition funnel. Dry dimethylsulfoxide (DMSO) (550 g) was added and the mixture was stirred and warmed to 60 °C as 1,2-dibromotetrafluoroethane (537 g, 2.06 mole) was added slowly. (No appreciable reaction was observed at lower temperatures.) Reaction temperature is maintained at 65 °C to 70 °C for two hours after addition is complete. The mixture was then heated to 90 °C and allowed to cool overnight.

Product was isolated by extracting the mixture with 500 mL of water to remove salts and DMSO. The product separated as an orange oil which was washed with water to remove residual DMSO. (The upper aqueous layer was extracted with methylene chloride, and the methylene chloride solution was evaporated to yield about 40 g of product which was added to the rest of the product prior to the water washes.) The product (623 g) was distilled at 85 °C 40 Pa (0.3 mm Hg) to yield 561 g of colorless oil, 85 percent yield. The product was identified by 19F NMR, 1H NMR, and IR spectra.

To form a salt suitable for formation of the perfluorovinyl ether, another sample of methyl 4-(2-bromotetrafluoroethoxy)benzoate (66.25 g, 0.2 mole) was weighed into a 4-necked 500 ml round-bottomed flask fitted with a condenser, thermometer, mechanical stirrer, and heating mantle. Methanol (300 mL) and sodium hydroxide (8.05 g, 0.2 mole) were added to form a fixture which was stirred and heated to reflux for three hours. A sodium carboxylate formed and began to precipitate early in the reaction and was gelled into an almost solid mass after 1.5 hours. The mass was allowed to settle overnight and the solvent was then removed by rotary evaporation.

The sodium carboxylate was dissolved in warm water. A warm solution of zinc acetate (26.35 g, 0.12 mole) in 40 mL of water was added to precipitate the carboxylate as the zinc salt. The salt slurry was then cooled, and the zinc salt was filtered from the solution and dried under vacuum to yield 65.6 g (94 percent yield).

The dried zinc salt was transferred to a dry 4-necked 500 mL round-bottomed flask containing zinc metal (1.7 mm (10 mesh), 13.0 g, 0.198 mole). Dry glyme (160 mL) was added by a canula and the flask

was fitted with a condenser, mechanical stirrer, and thermometer. The mixture was stirred and heated to reflux under nitrogen overnight. The mixture was acidified by the addition of 18 mL of concentrated HCl, concentrated by rotary evaporation, and then partitioned between methylene chloride and water. The methylene chloride solution of the acid was dried over magnesium sulfate, filtered and concentrated to yield 40.02 g of 4-trifluoroethenyloxybenzoic acid as white crystals (97.6 percent yield, melting point 139 °C to 140 °C). The product 4-trifluoroethenyloxybenzoic acid was identified by 19F NMR, 1H NMR, and IR spectra.

To form the 4-trifluoroethyloxybenzoyl chloride, 4-trifluoroethenyloxybenzoic acid (79.4 g, 0.36 mole) was transferred to a 1-liter round-bottomed flask. Dry methylene chloride (250 mL) was added, and the resulting mixture was stirred under nitrogen as oxalyl chloride (62.5 g, 0.49 mole) was added. The mixture was stirred overnight and then concentrated by rotary evaporation. The brown liquid was distilled at 60 °C to 65 °C 26.6 Pa (0.2 mmHg) to yield 82.94 g of colorless liquid (97.4 percent yield). The product was identified by 19F NMR, 1H NMR, and IR spectra.

To cap an oligomer, a low molecular weight polycarbonate oligomer (2000 MW) terminated with bisphenol A groups (7.5 g, about 7.8 x $10^{-3}$ mole of phenolic OH) was weighed into a 100 mL flask with the trifluoroethenyloxybenzoyl chloride (1.84 g, 7.8 x $10^{-3}$ mole). Dichloromethane (30 mL) was added to dissolve the oligomer, and the mixture was stirred as triethylamine (0.81 g, 8 x $10^{-3}$ mole) was added via syringe. A fine white precipitate forms in the mixture almost immediately. Dichloromethane was added to dissolve the precipitate, forming a dichloromethane solution which was extracted with water to remove triethylamine hydrochloride. The dichloromethane solution was dried over 4 x $10^{-10}$m (4A) molecular sieves, and concentrated to yield 9.06 g (100 percent yield) of oligomer capped with trifluoroethenyloxy benzoyl groups. Structure was verified by 19 F NMR (trifluorovinyl ether pattern), H-NMR(2 protons of the aromatic benzoate were shifted downfield to 8-8.3 ppm from the aromatic polycarbonate protons), and FT-IR (C=O stretch at 1739 $cm^{-1}$, distinct from the C=O stretch of polycarbonate at 1774 $cm^{-1}$).

A sample of the capped oligomer was heated to 300 °C in a DSC apparatus to effect chain extension. The sample was cooled and reheated to determine the Tg, which was observed at 140.4 °C (representative of high molecular weight polycarbonate). For comparison, a sample of the uncapped oligomer heated to 300 °C, cooled, and reheated, exhibited a Tg of only 106.8 °C. The increase of 33.6 °C in the Tg was attributed to the production of high molecular weight polycarbonate through the thermal cyclodimerization of the trifluorovinyl ether groups.

EXAMPLE 15: REACTION OF 4,4'-BIPHENOL AND TRIFLUOROVINYLOXYBENZOYL CHLORIDE

Dihydroxybiphenyl (0.7888 g, 0.00423 mole) was placed in a dry 250 mL round bottom flask with a magnetic stirring bar. The flask was capped with a rubber septum. Dry methylene chloride (25 mL) and trifluorovinyloxybenzoyl chloride as prepared in Example 14 (2.000 g, 0.00846 mole) were each added to the flask via syringe. The mixture was stirred as triethlyamine (0.86 g, 0.0085 mole) was added dropwise. The mixture was stirred at room temperature for 2 hours, then filtered. A white precipitate was obtained and washed several times with methylene chloride to remove residual triethylamine hydrochloride. A white crystalline product was obtained and had a melting point of 225 °C to 228 °C. Qualitative solubility tests indicated that this product was nearly insoluble in methylene chloride, acetone, acetonitrile, hexane, methanol, water and benzene, only slightly soluble in hot tetrahydrofuran, and moderately soluble in carbon tetrachloride.

Infrared analysis (using a potassium bromide KBr pellet) gave the following spectrum (reported in $cm^{-1}$): 1830, indicative of a trifluorovinyl group: 1723, indicative of a benzoate ester; 1600 and 1495, indicative of aryl carbon-carbon double bond; 1315 and 1267, indicative of carbon-fluorine bonds.

Thermal analysis (DSC) of the monomer indicated a crystalline melt beginning at 223 °C, followed immediately by a slight exotherm as the monomer underwent polymerization. A second scan of the sample showed no thermal activity up to and including 350 °C.

The melted monomer exhibited possible liquid crystalline behavior during it's short lived melt phase. As viewed under a cross-polarized light microscope, the melted monomer phase (at 230 °C) exhibited birefringence suggestive of liquid crystalline behavior, followed by rapid polymerization to a crystalline solid. This solid did not melt, but underwent discoloration and apparent decomposition when heated in air at temperatures above 400 °C.

EXAMPLE 16: PREPARATION OF POLYESTERS FROM 1,2-BIS(4-CHLOROFORMYLPHENOXY)-HEXAFLUOROCYCLOBUTANE BY SOLUTION POLYMERIZATION

Methyl p-hydroxybenzoate was converted to its potassium salt by reaction with a stoichiometric amount of potassium hydroxide in methanol. The salt was isolated by evaporation and dried under vacuum. The dried salt was slurried in an equal weight of dry dimethyl sulfoxide. The mixture was stirred and heated to about 50 °C and a slight excess of 1,2-dibromotetrafluoroethane was added slowly. The reaction temperature was maintained at 60 °C to 70 °C. An efficient condenser was necessary to condense the dibromotetrafluoroethane. After addition was complete, the mixture was warmed for an additional hour, cooled and poured into an equal volume of water. The product (methyl 4-(2-bromotetrafluoroethoxy)-benzoate) separated as a brown oil which was distilled under vacuum (85 °C to 90 °C, 39.9 Pa (0.3 mmHg)) to yield a colorless oil (85 to 95 percent yield).

The bromotetrafluoroethylether was dehalogenated by combining it with a stoichiometric amount of granular zinc in glyme and refluxing overnight. After removal of the glyme by evaporation, the product, methyl 4-trifluoroethenyloxybenzoate, was distilled under vacuum (85 °C to 90 °C 1064 Pa to 1330 Pa (8-10 mmHg), 85 to 100 percent yield).

The methyl 4-trifluoroethenyloxybenzoate was cyclodimerized by heating at 195 °C for several hours. The dimerized product was isolated by distillation (135 °C to 150 °C 2.3 Pa (0.025 mmHg), 97 percent yield, with the remainder being unreacted vinyl compound). The overall yield from methyl p-hydroxybenzoate was 80 percent.

The dimer was saponified to the diacid with 2.1 molar equivalents of sodium hydroxide in methanol. Upon acidification with concentrated hydrochloric acid the diacid precipitates and was filtered from the liquidas an insoluble white powder with a melting point above 300 °C. Yields were quantitative. The diacid was converted to the diacid chloride by slurrying it in approximately a 6 molar equivalent of thionyl chloride and warming the mixture to 50 °C to 75 °C. The product diacid chloride was soluble in dichloromethane and was purified by dissolving the crude reaction product in dichloromethane and filtering the diacid chloride solution from unreacted diacid (which was insoluble). The product was identified by 19FNMR, HNMR and infrared (IR) spectra. IR 1790, 1755 $cm^{-1}$ (C=O) no $CO_2H$ absorption.

Bisphenol AP (1,1-bis(4-hydroxyphenyl)-1-phenyl ethane) (6.14 g, 21.1 mmole) was transferred to a dried resin kettle (a reaction vessel with a large top that clamps onto the vessel) along with 50 mL of dry dichloromethane. The mixture was stirred under nitrogen as triethylamine (5.9 mL, 42.2 mmole) was added via syringe. The solution was stirred and cooled in a water bath as a solution of 1,2-bis(4-chloroformylphenoxy)-hexafluorocyclobutane (10.0g, 21.1 mmole) in dichloromethane (50 mL) was added via syringe. The mixture was allowed to stir overnight under nitrogen to form a polymer in solution.

The polymer was then capped by adding 0.25 mL of 4-trifluoroethenyloxybenzoyl chloride (as prepared in Example 14) to the solution with stirring. The solution was diluted with 200 mL of dichloromethane and washed with water to remove triethylamine hydrochloride until a sample of the water washes added to a 5 percent silver nitrate solution produced no silver chloride precipitate. The polymer solution was then poured into a glass dish, and the dichloromethane was allowed to evaporate overnight, leaving a clear, tough film, which was dried under vacuum at 140 °C and was weighed. (Yield = 14.58 g, 99.9 percent)

EXAMPLE 17: PREPARATION OF POLYESTERS FROM 1,2-BIS(4-CHLOROFORMYLPHENOXY)-HEXAFLUOROCYCLOBUTANE BY EMULSION POLYMERIZATION

Bisphenol AP (10.51 g, 36 mmole) is transferred to a blender container along with water (200 mL), 50 percent aqueous sodium hydroxide (6.6 g, 82 mmole) and benzyltrimethylammonium chloride (2 g, 6.5 mmole, 60 percent aqueous solution). Agitation was supplied by a blender plugged into and having its speed controlled by a Variac. The mixture was agitated at 25 to 30 percent power until the bisphenol AP dissolves.

1,2-bis(4-chloroformylphenoxy)hexafluorocyclobutane (prepared as in Example 16) (17.12 g, 36 mmole), was dissolved in 70 mL of dichloromethane to form a diacid chloride solution and was chilled in an ice bath. Dichloromethane (25 mL) was added to the blender, which was agitated at 30 percent power for 2 minutes, at which time the chilled diacid chloride solution was added to the blender over a period of 20 seconds to form an admixture. The container in which the diacid chloride mixture was chilled was rinsed with 30 mL of dichloromethane, which was added to the admixture. The admixture was agitated at 40 percent power for 12 minutes; then 1.2 mL of benzoyl chloride was added. The admixture was agitated for an additional 2 minutes. Then agitation was stopped and layers are allowed to separate. An aqueous layer was decanted, and a lower, dichloromethane layer, was agitated with 200 mL portions of deionized water until a sample of

the water tested negatively for chloride ion.

Addition of an equal volume of isopropyl alcohol to the volume of dichloromethane layer precipitated a polymer from the dichloromethane solution as a thick, viscous mass. The polymer was allowed to air dry, then redissolved in dichloromethane to form a clear solution and poured into a glass dish. Evaporation of the dichloromethane overnight yielded a tough clear film which was dried under vacuum at 140 °C. Weight of recovered polymer was 24.56 g, 98 percent yield.

EXAMPLES 18-23: POLYESTERS PREPARED USING 1,2-BIS(4-CHLOROFORMYLPHENOXY)-HEXAFLUOROCYCLOBUTANE

The process of Example 16 for the solution preparations and the process of Example 17 for the emulsion preparations were repeated using the following bisphenols with the results indicated in the following Table III, which includes polymers of Examples 16 and 17 for comparison:

Table III

| Ex. No. | Bisphenol ** | Yield (%) | Tg (°C) | Molecular Weight | ppPolymerization Method | Dielectric Constant/dissipation Factors at 1 KHz |
|---|---|---|---|---|---|---|
| 18 | Bisphenol A | 100 | 137 | 47000 | solution | 2.93/0.0031 |
| 16 | Bisphenol AP | 99.9 | 178 | 60000 | solution | 3.05/0.0042 |
| 19 | Bisphenol AF | 97 | 160 | 66000 | solution | 2.94/0.0036 |
| 20 | 4,4'-Biphenol | 98 | >400 | -- | solution | * |
| 21 | Hydroquinone | 99 | >400 | -- | solution | * |
| 22 | Bisphenol A | 100 | -- | 64000 | emulsion | -- |
| 17 | Bisphenol AP | 98 | 185 | 104000 | emulsion | -- |
| 23 | Bisphenol AF | 100 | -- | 79000 | emulsion | -- |

* These materials were highly crystalline and precipitated from the reaction mixture. Molecular weights could not be determined by GPC due to insolubility.

** Bisphenol AF is 2,2-bis(4-hydroxyphenyl)-hexafluoropropane; bisphenol A is 2,2-bis(4-hydroxyphenyl)propane.

Tensile and flexural strength were determined for a sample of the bisphenol A polymer prepared by solution polymerization (Example 18).
Tensile strength was 44.5 x $10^3$ kPa (6460 pounds/square inch).
Flexural strength was 21.1 x $10^3$ kPa (3060 pounds/square inch).
Flexural Modulus was 22.8 x $10^5$ kPa (330,000 pounds/square inch).

EXAMPLE 24: POLYMERIZATION OF 1,2-BIS(4-ETHYNYLPHENOXY)HEXAFLUOROCYCLOBUTANE

p-Ethylphenol was dissolved in methanol to form an admixture. A methanolic solution of one equivalent of potassium hydroxide was added to the stirring admixture. The admixture was cooled to maintain a temperature below 40 °C. Stirring and cooling was maintained for about 15 minutes after addition was complete.

The methanol was then removed by rotary evaporation and a resulting wet salt was transferred to a suitable container and dried under vacuum at 100 °C to 140 °C to produce a dry salt. The dry salt was transferred to a dry flask and an equal volume of dry DMSO was added to form a slurry. The flask was fitted with a mechanical stirrer, thermometer, efficient condenser, and pressure-equalizing addition funnel.

The salt slurry was stirred and cooled to less than 20 °C as a slight excess (1.1 equivalents) of 1,2-dibromotetrafluoroethane is added slowly. The reaction was heated to 60 °C for about 2 hours after addition was complete.

The 2-bromotetrafluoroethyl ether was isolated by pouring into an equal volume of water. The ether separated as a lower layer of oil and was purified by vacuum distillation.

4-Trifluorovinyloxy-1-ethylbenzene was synthesized by combining the 2-bromotetrafluoroethyl ether with granular zinc in dry glyme and refluxing at 85 °C to 90 °C with stirring overnight.

After completion of the reaction, the precipitated zinc salts were removed by centrifugation. The glyme was removed by rotary evaporation and the product, 4-trifluorovinyloxy-1-ethylbenzene, was purified by vacuum distillation.

4-Trifluorovinyl-1-ethylbenzene was cyclodimerized by heating to 180 °C to 195 °C for 8 hours. Low boiling impurities and unreacted perfluorovinyl compound were removed by vacuum distillation. The product 1,2-bis(4-ethylphenoxy)hexafluorocyclobutane was distilled under vacuum (110 °C 6.6 Pa (0.05 mmHg)) 40 percent yield from 4-ethylphenol.

A 5 g sample of 1,2-bis(4-ethynylphenoxy)hexafluorocyclobutane (as prepared in Example 17 was thermally cured at 180 °C for 3 hour followed by a postcure at 250 °C for 30 minutes. The density of the polymer was 1.34 g/$cm^3$ Dielectric constant was 2.6 at 1 KHz.

DSC analysis of 1,2-bis(4-ethynylphenoxy) hexafluorocyclobutane indicated a broad exotherm starting at 160 °C and ending at 280 °C with delta H = 632 Joules/g. The initially colorless oil turned into a clear, dark red solid which was analyzed by DSC, TMA, and TGA (Thermogravimetric analysis). TGA (20 °C/min., nitrogen sweep) of the cured sample shows: 2 percent loss at 400 °C, 5 percent loss at 470 °C, 35 percent loss at 580 °C, and 80 percent loss at 900 °C. No Tg was observed up to 350 °C by DSC or TMA.

EXAMPLE 25: SYNTHESIS AND POLYMERIZATION OF 1,1,1-TRIS(4-TRIFLUOROETHENYLOXYPHENYL)-ETHANE

Potassium hydroxide (678.72 g, 10.28 mole, 85 percent) was dissolved with cooling into 630 mL of water in a 5 liter round-bottomed flask fitted with a thermometer and stirrer. Methanol (400 mL) was added and the mixture was stirred under nitrogen in an ice bath as a solution of THPE [1,1,1-tris(4-hydroxyphenyl)-ethane] (1051 g, 3.43 mole) in 1650 mL of warm methanol (40 °C) was added. Methanol was then removed from the resulting solution of the potassium salt of THPE in water/methanol by distillation under reduced pressure (18.7 kPa (140 mm Hg), 55 °C to 60 °C) with addition of a total of 1245 mL of water to make up for losses and to obtain a solution of THPE potassium salt in water with the consistency of light syrup. The potassium salt of THPE was isolated by evaporation of this water solution (48 percent solids by weight) in a drum dryer. The resulting dried THPE salt contained about 4 percent water by weight. The dried THPE salt was transferred to a 12 L round-bottomed flask fitted with a condenser and water trap along with 5 L or dimethylsulfoxide (DMSO) and 1.5 L of toluene. This slurry was stirred and heated to reflux under nitrogen to remove residual water by azeotroping it with toluene. After 6 hours of reflux no further water collection was seen in the water trap and the water trap was replaced with a Soxhlet extractor containing dry sodium sulfate. The mixture was refluxed through the Soxhlet extractor for a total of 5.5 h with the sodium sulfate being replaced with an equivalent amount of dry sodium sulfate once at the halfway point, during this process. About 1 L of toluene was then removed by distillation and the remaining mixture was cooled to

18 °C by means of an internal cooling coil containing either tap water or chilled glycol as needed. The temperature of the stirring mixture was maintained at 16 °C to 18 °C by means of this internal cooling coil as 1,2-dibromotetrafluoroethane (3000 g, 11.54 mole, 1382 mL) was added over 1 hour and the resulting mixture was stirred at 18 °C for 36 hours.

The reaction was worked up by pouring the mixture into an equal volume of cold water with stirring and then allowing the layers to separate. The lower layer containing the product was drained and the upper aqueous layer was washed with hexane. The hexane wash was combined with the product layer and evaporated under reduced pressure to remove solvents. The crude product solidified into a crystalline mass melting at ca. 100 °C. This mass was melted and distilled at reduced pressure (190 °C to 195 °C, 6.7 Pa (0.05 mm Hg)) to yield the product, 1,1,1-tris(4-(2-bromotetrafluoroethoxy)phenyl)ethane, as a yellowish viscous oil which crystallizes upon standing. The yield of this reaction was 88.1 percent and the product was 93 percent pure with the remaining 7 percent being the byproduct 1,1-bis(4-(2-bromotetrafluoroethoxy)-phenyl)-1-(4-(1,1,2,2-tetrafluoroethoxy)phenyl)ethane which resulted from the replacement of one of the bromine atoms of the product with a hydrogen atom. The byproduct may be removed by melting the distilled crystalline mass and pouring it into an equal volume of methanol with stirring. The product preferentially crystallizes into fluffy white needles which can be filtered from the methanol solution. Alternatively, the 93 percent pure product may be used directly in the dehalogenation reaction.

The crystalline 1,1,1-tris(4-(2-bromotetraflucroethoxy)phenyl)ethane is found to have the following properties:

White needles, melting point 110 °C;

F-19 NMR: delta -10.4 (t, J = 6 Hz, which is attributed to $-CF_2Br$), 7.5 (t, J = 6 Hz, which is attributed to $OCF_2$);

H-NMR: delta 2.2 (bs, 3 H), 7.1 (bs, 12 H); and

IR: cm-1 1501, 1329, 1312, 1206, 1173, 1126, 1100, 1012, 929, 784.

Granular zinc (213.3 g, 3.26 mole, 0.85 to 1.7 mm (10-20 mesh)) was weighed into an oven-dried 3 L round-bottomed flask. The flask was then fitted with a stirrer, thermometer, septum, and a 1 L pressure-equalizing addition funnel containing 1,1,1-tris(4-(2-bromotetrafluoroethoxy)phenyl)ethane (833.7 g, .989 mole, 97 percent purity). Dry diglyme (1045 g, 1115 mL) was added to the flask via canula and also to the addition funnel (384 g, 410 mL). The septum was replaced with a stopper, and the zinc/diglyme slurry was stirred and heated to 115 °C as the contents of the addition funnel were heated by means of heating tape to ca. 80 °C to form a homogeneous solution. The solution of 1,1,1-tris(4-(2-bromotetrafluoroethoxy)phenyl)-ethane in diglyme was added slowly to the hot stirring zinc/diglyme slurry over 2 h, maintaining the reaction temperature at 115 °C to 125 °C by varying both heating rate and rate of addition. After addition was complete the mixture, which now contains precipitated zinc salts, was heated at 120 °C to 125 °C for an additional 5 h. The mixture was allowed to cool and the precipitated zinc salts were removed by centrifugation. Ethyl acetate was used to rinse the flask.

The resulting diglyme solution of the tris-perfluorovinyl ether product, 1,1,1-tris(4-trifluoroethenyloxyphenyl)ethane, was concentrated under reduced pressure by rotary evaporation and the resulting mixture of product and zinc salts was slurried in hexane and flushed through a 15.2 cm (6 inch) by 15.2 cm (6 inch) column of neutral alumina commercially available from Aldrich Chemical Company under the trade designation Brockmann I. The product was isolated by evaporation of the hexane solution under reduced pressure. The product was isolated as a colorless oil (85 to 90 percent yield, 89 to 79 percent purity). The remainder of the material (7 to 10 percent) was a bis-perfluorovinyl ether, 1,1-bis(4-trifluoroethenyloxy)-phenyl)-1-(4-(1,1,2,2-tetrafluoroethoxyphenyl)ethane.

The bis-perfluorovinyl ether was removed by countercurrent extraction to obtain up to 99.9 percent pure tris-perfluorovinyl ether. Some solvent mixtures suitable for the separation were hexane/acetonitrile, hexane/dimethyl sulfoxide, and hexane/dimethylformamide. A countercurrent extractor was constructed from PFA (perfluoroalkoxy) tubing (2.54cm (1 inch) inside diameter, 1.22 m (4 feet) length) packed with stainless steel packing, commercially available from Metex Corp. under the trade designation Goodloe™, two electronic pumps commercially available from Pennwalt Corp. under the trade designation CHEMPULSE™ (Pennwalt model #45-050/KIM), two solvent reservoirs filled respectively with mutually saturated hexane and acetonitrile, and two receivers.

A sample of impure tris-perfluorovinyl ether (180.8 g, 93.5 percent tris-, 6.5 percent bis-perfluorovinyl ether; purity as determined by gas chromotography corresponding to mole percent) was diluted with 1500 mL of hexane to make an approximately 10 percent (by volume) solution. The pumps were started, with hexane pumped into the bottom of the column (above the acetonitrile take-off) and acetonitrile pumped into the top of the column (below the hexane take-off). The flow rates were adjusted to 90 mL/minute for hexane and 45 mL/minute for acetonitrile. The hexane reservoir was allowed to drain until nearly empty, and the

solution of impure tris-perfluorovinyl ether was transferred to the reservoir. After this was transferred onto the column, the reservoir was filled again with 1500 mL of hexane (saturated with acetonitrile). This was allowed to pump through the column and into the product reservoir to ensure complete washing of the product from the column. The hexane solution which exited the column contained tris-perfluorovinyl ether. (99.5 percent purity, 49 percent recovery). The acetonitrile solution which exited the column contained the remainder of the product (88.35 g, 85.5 percent tris- and 14.5 percent bis-perfluorovinyl ether) and was concentrated by rotary evaporation, rediluted in hexane (750 mL) and purified by running it through the countercurrent extractor to give 50.55 g of tris-perfluorovinyl ether (98.2 percent purity). The acetonitrile solution from this pass contained 25.7 g of material which was 71 percent tris and 28.5 percent bis-perfluorovinyl ether. The ether was found to have the following properties:
1,1,1-tris(4-trifluoroethenyloxyphenyl)ethane:
Colorless, mobile oil
F-19 NMR: delta 42.7 (dd, J(cis) = 60 Hz, J(gem) = 100 Hz, which was attributed to =CF (terminal vinyl fluorine), cis to F 49.3 (dd, J(trans) = 120 Hz, J(gem) = 100 Hz, which was attributed to =CF, trans to F 55.3 (dd, J(cis) = 60 Hz J(trans) = 120 Hz, which was attributed to OCF groups);
H-NMR: delta 2.1 (bs, 3 H), 6.95 (bs, 12 H); and
IR: cm-1 1830 which was attributed to ($CF=CF_2$) groups, 1500, 1313, 1273, 1205, 11781, 1138, 1011.
The 1,1,1-tris(4-trifluoroethenyloxyphenyl)ethane polymerized at 140°C to 350°C, with a peak exotherm at 235°C as determined by differential scanning caloimetry (DSC).

A polymer was prepared by degassing a 9 g sample of the 93.5 percent pure product obtained before purification. 1,1,1-tris(4-trifluoroethenyloxyphenyl)ethane under vacuum until no visible bubbles form at room temperature; then heating the sample to 150C to 160°C for 2 hours, then followed by heating at 240°C for 30 minutes. The sample was then allowed to cool to room temperature. The sample was a solid which was then chipped out of its container and ground into a powder using mortar and pestle. The powder was poured into a mold measuring about 10.2 by 12.7 by 0.008 cm (4 by 5 by 1/32 inches), put in a press, and compression molded at 240°C and 68.9 x $10^3$ kPa (10,000 psig (pounds per square inch on the gauge)) for 15 minutes. A resulting plaque was removed without cooling and was observed to be clear, flexible and colorless.

The polymer was found to have a Tg of 282.1°C as measured by thermomechanical analysis (TMA) on a mettler TA 3000

EXAMPLE 26: POLYMERIZATION OF 1,1,1-TRIS(4-TRIFLUOROETHENYLOXYPHENYL) ETHANE

A 30 g sample of the material prepared in Example 25 which is 99.5 percent pure 1,1,1-tris(4-trifluoroethenyloxyphenyl) ethane is filtered and poured into a vertical casting mold measuring 7.6X10.2X0.3cm (3 by 4 by 1/8 inches). The mold was placed in a vacuum oven at 140°C to 160°C for a period of 2 hours after which, the heat was increased to 240°C to 250°C for a period of 1 hour. The heat was turned off and the mold allowed to cool to room temperature. The molded polymer was removed and found to have the following properties:
Colorless, transparent, Tg (as measured by Thermomechanical Analysis on a Mettler TA 3000) = 300°C; Dielectric Constant (as measured by the procedure of ASTM D150-87) = 2.45 (1 MHz) Dissipation Factor (as measured by the procedure of ASTM D150-87) = 0.0005 (1 MHz). Upon postcuring by heating twice from 250°C to 360°C at a rate of 10°C per minute; a Tg of 414°C was obtained.

EXAMPLE 27: POLYMERIZATION OF 1,1,1-TRIS(4-TRIFLUOROETHENYLOXYPHENYL) ETHANE

The procedure of Example 25 was repeated obtaining samples of 1,1,1-tris(4-trifluoroethenyloxyphenyl) ethane having a purity of 97 mole percent after having been flushed through alumina using hexane.

A sample of the 97 percent pure 1,1,1-tris(4-trifluoroethenyloxyphenyl) ethane was poured into a compression mold and heated to 160°C for a period of 2 hours then 240°C for a period of 30 minutes, after which it was cooled to 25°C and removed from the mold. The resulting polymer was found to have a Tg of 286.1°C and a dielectric constant (as measured by the procedure of ASTM D15D-87) of 2.55 and dissipation factor (as measured by the procedure of ASTM D15D-87) of 0.001 (1 Mega Hertz (MHz)).

EXAMPLE 28: POLYMERIZATION OF 1,1,1-TRIS(4-TRIFLUOROETHENYLOXYPHENYL) ETHANE

The procedure of Example 25 is repeated obtaining samples of 1,1,1-tris(4-trifluoroethenyloxyphenyl) ethane having the following purities after the indicated steps:

Table IV

| Step No. | Step | purity (mole percent * | Tg °C |
|---|---|---|---|
| 6 | catalyst and solvent (hexane) removal | 89.6 | 276 |
| 7 | counter current extraction using hexane/acetonitrile, first cut in hexane | 99.8 | 300 |
| 8 | recover* from acetonitrile without further purification | 73.6 | 242 |
| 9 | acetonitrile sample after purifiaction by second extraction in hexane | 85.7 | 251 |
| 10 | recovery from acetonitrile extraction of step 8 | 50 | 178 |

*as determined by gas chromatogrphay

A sample of each of the above materials were poured into a pan and put into a vacuum oven at 140 °C for a period of 2 hours, after which the temperature was raised to 240 °C for a period of 1 hour. The pan was removed from the oven and the resulting polymer was cooled to room temperature and removed from the pan. Using TMA using a Mettler TA 3000 system, the samples were found to have the Tg's indicated in Table IV.

The samples of steps 8 and 10 were observed to soften indicative of thermoplastic character, and the samples of steps 6, 7 and 9 were observed to undergo a change in rate of expansion indicative of thermoset character.

These examples show that various purities of tris-perfluorovinyl monomers are obtained using various purification methods and that Tg increases with percentage of polyfunctional monomer. Counter current extraction is particularly useful for removing such impurities as bis-perfluorovinyl ethers. With such extractions, tris-perfluorovinyl monomers exceeding 99.9 percent in purity are obtained and are particularly useful, for instance in electronics uses. Generally such counter current solvent extractions require two solvents which are substantially immiscible one of which preferentially dissolves the desired product. Solvent combinations useful for the separation useful in the practice of the present invention include solvents for the desired monomer paired with solvents in which the monomer is less soluble, but at least some of the impurities are more soluble. Such solvent pairs include hexane/acetonitrile, hexane/dimethylsulfoxide, and hexane/dimethylformamide.

Alternatively, the monomers are suitable for polymerization after preparation without purification, preferably with removal of solvent(s) used in preparation. Such monomers in combination with by-products of their preparation are suitable, for instance, for applications wherein lower Tg's are suitable such as objects.

EXAMPLE 29: CROSSLINKING OF 4,4'-BIS(TRIFLUOROVINYLOXY)BIPHENYL

After the preparation and polymerization of 4,4-bis(trifluorovinyloxy) biphenol in Example 1 and 2, a sample plaque of the biphenyl perfluorocyclobutyl ether polymer (12 cm x 8 cm x 1 cm) was placed inside a baking dish in a vacuum drying oven and was cured under vacuum at 310 °C for 20 hours. The sample was removed and a coupon was cut (6.0 cm x 1.2 cm x 0.33 cm) for dynamic mechanical analysis. The analysis showed a Tg of 175 °C with no complete melt occurring, as was evidenced by maintenance of a storage modulus up to and including 344 °C. This cured (crosslinked) polymer also did not dissolve in THF but swelled into a gel.

A sample of the thermoplastic biphenyl perfluorocyclobutyl ether polymer was placed in a Rheometrics RMS-605 Mechanical Spectrometer using 25 mm parallel plates with a 1 mm gap. Using a 10 percent strain, storage and loss moduli were measured from 0.1 to 100 radians per second, measuring ten different frequencies per decade of frequency, every 15 minutes. Isothermal measurements were carried out according to the technique of H.H. Winter and F. Chambon, J. Rheology, 30(2), 367-382 (1986) except that the gel points were measured at 320 °C and 360 °C. The log of tan delta (G''/G'), that was the logarithm of the ratio of the loss modulus (G'') to the storage modulus (G'), was plotted against time for various frequencies. A gel point was indicated by convergence of the various frequency dependent tan deltas in the plot at 15 minutes. A second experiment was run under similar conditions, but at 320 °C isothermal; a plot of tan delta against time indicated a gel point at 80 minutes. Furthermore, samples after being heated

beyond the gel points were not soluble in tetrahydrofuran. Thus, the system appears to crosslink in 15 minutes at 360 °C and in 80 minutes at 320 °C. Crosslinked samples exhibited some reddish brown or yellow color.

Physical property determinations on samples of the thermoplastic (uncrosslinked) polymer and on the crosslinked polymer showed significant differences as indicated in Table V below:

Table V

| Physical Property Comparison | | |
|---|---|---|
| | Thermoplastic Polymer | Crossinked Polymer |
| Tensile Strength[1] | 37.3 x $10^3$ kPa (5,500 psi) | 49.6 x $10^3$ kPa (7,200 psi) |
| Tensile Modulus[1] | 13.8 x $10^5$ kPa (200,000 psi) | 17.6 x $10^5$ kPa (255,000 psi) |
| Percent Elongation[1] | 12 % | 4% |
| Flexural Strength[2] | 74.5 x $10^3$ kPa (10,800 psi) | 60.0 x $10^3$ kPa (8,700 psi) |
| Flexural Modulus[2] | 16.1 x $10^5$ kPa (234,000 psi) | 21.7 x $10^5$ kPa (315,000 psi) |
| Dielectric Constant[3] | 2.57 (10kHz) | 2.59 (10 kHz) |
| Dissipation Factor[3] | 0.0004 (10 kHz) | 0.0006 (10 kHz) |

[1.] As determined by the procedures of ASTM D882-83.
[2.] As determined by the procedures of ASTM D790-81.
[3.] As determined by the procedures of ASTM D150-87.

The data in Table V shows that crosslinking increases tensile strength and modulus as well as flexural modulus while reducing elongation without substantial change in electrical properties.

EXAMPLE 30: CROSSLINKING OF 9,9-BIS(4'-TRIFLUOROVINYLOXY]PHENYL)FUORENE

After preparation and polymerization of 9,9-bis(4'[trifluorovinyloxy]phenyl)fluorene, as in Example 3, a sample of the 9,9-bis(4'-oxyphenyl)fluorene perfluorocyclobutyl ether polymer was placed in the Rheometrics RMS-605 Mechanical Spectrometer using the conditions of Example 29. A plot of the tan delta against time for a 360 °C isothermal experiment indicated a gel point in less than 10 minutes. A similarly run experiment at 320 °C isothermal indicated a gel point in 35 minutes.

These gel points were indicative of curing of the samples into crosslinked polymer systems. Thus, the system appeared to crosslink in less than 10 minutes at 360 °C and in 35 minutes at 320 °C. Samples of the polymer heated to the gel point were insoluble in acetone, dichloromethane and tetrahydrofuran. The Tg was measured by differential scanning calorimetry to be 240 °C.

EXAMPLE 31: PREPARATION, POLYMERIZATION AND CURING OF 2,2-BIS((4-PERFLUOROVINYLOXY)-PHENYL)PROPANE

The procedure for preparing the diperfluorovinyl compound of Example 1 was followed except that smaller scale equipment was used with about half the amounts of solvent for the various steps using 100.0 g, 0.44 mole of para-bisphenol A in place of the 4.4'-dihydroxybiphenyl; 59.0 g, 0.89 mole of potassium hydroxide pellets: 240 g, 0.92 mole of 1,2-dibromotetrafluoroethane; and 25.0 g, 0.38 mole of granular zinc. The toluene mixture was heated to reflux (125 °C). Water was removed by azeotropic distillation for a total of 48 hours, without cooling after 24 hours. Before addition of the 1,2-dibromotetrafluoroethane, the toluene was dried by refluxing through the extractor for 20 hours. After cooling to room temperature the reaction flask was cooled to 18 °C in an ice bath. The 1,2-dibromotetrafluoroethane was added at a rate that maintained a reaction temperature of 18 °C to 22 °C. Then the mixture was allowed to warm to room temperature, and was heated slowly to 50 °C and stirred at 50 °C for 6 hours.

A crude reaction mixture formed and was filtered. The filtrate was evaporated thoroughly leaving a residue which was chromatographed on neutral alumina (0.08 to 0.315 mm (50 to 200 mesh)) using hexane as eluent to provide 113.9 g (44 percent yield) of a product having a GC/MS with peaks at the following mass to charge ratios (m/e): 587 (4.6%); 585 (9.2%); 583 (5.0%); 572 (44.5%); 570 (100%); 568 (52.0%);

397 (11.3%); 395 (11.0%); 315 (27.1%); 313 (28.2%); 299 (28.0%); 297 (30.4%); 181 (31.1%), 179 (37.6%); 167 (29.1%); 165 (37.8%); 131 (33.6%); 129 (36.9%); 115 (32.4%); 101 (35.7%); 91 (31.3%); 77(33.5%) consistent with a bisphenol A bis(2-bromotetrafluoroethyl) ether, 99+ percent pure by GC/MS analysis.

After reaction with the granular zinc at 105°C, the bisphenol A bis(2-bromotetrafluoroethyl) ether (103.4 g, 0.176 mole) was placed in a 100 mL dropping addition funnel and added at a rate that maintained a reaction temperature of 105°C to 108°C. When the addition was complete the mixture was stirred at 108°C for 2.5 hours, then cooled to room temperature. The mixture was centrifuged to remove the solids; the precipitate was separated and washed with acetone and again separated by centrifuging. The liquid portions were combined and evaporated leaving a residue which was chromatographed through a neutral alumina column using hexane as an eluent to provide 42.8 g (63 percent yield) of a product having a GC/MS with peaks at the following mass to charge ratios (m/e): 388 (17.5%); 374 (20.0%); 373 (100%); 276 (30.4%); 215 (46.7%); 199 (12.8%); 179 (24.8%); 178 (50.0%); 152 (15.6%); 118 (24.0%); 117 (18.3%); 115 (17.1%); 102 (19.9%); 89 (23.5%); 77 (22.6%); 76 (29.9%) bisphenol A bis(trifluorovinyl) ether.

The bisphenol A bis(trifluorovinyl) ether monomer (13.6 g) was combined with 14.0 mL of Multifluor™ APF 215 solvent in a 100 mL 3-necked round bottom flask fitted with a mechanical stirrer and a nitrogen padded reflux condenser. Stirring was begun as the mixture was heated to reflux. The mixture was stirred at reflux for 5 hours, then allowed to cool to room temperature. A layer of polymer phase-separates to the top of the solvent. This polymer was removed and evaporated at 180°C under high vacuum (19.9 Pa (0.15 mmHg)) to remove residual solvent.

A sample of the polymer was placed in the Rheometrics RMS-605 Mechanical Spectrometer using the conditions used in Example 29. A plot of the tan delta against time for a 360°C isothermal experiment indicated a gel point in 50 minutes. A similar experiment at 320°C isothermal indicated that no gel point was reached by this polymer in the 150 minute time span of the experiment. These rheological experiments indicate that crosslinking of this polymer was much slower.

## Claims

1. A polymer having a backbone comprising aromatic hydrocarbyl groups, perfluorocyclobutane rings and non-carbon atoms.

2. The polymer of claim 1 wherein the non-carbon atoms are oxygen or sulfur.

3. The polymer of claim 1 which has a Tg of at least 25°C.

4. A process for preparing a polymer having a backbone comprising aromatic hydrocarbyl groups, perfluorocyclobutane rings and non-carbon atoms by:
   (a) contacting monomers having at least two dimerizable perfluorovinyl groups represented by Formula I:

   $CF_2=CF\text{-}X\text{-}R\text{-}(X\text{-}CF=CF_2)_m$

   wherein R represents an unsubstituted or inertly substituted aromatic hydrocarbyl group, each X is any group which links R and a perfluorovinyl group, and m is an integer of from 1 to 3; and
   (b) exposing the monomers to sufficient heat of from 40°C to 450°C such that a polymer containing perfluorocyclobutane rings is formed thermally.

5. The process of claim 4 wherein R has 6 to 50 carbon atoms and X is a group containing an oxygen or sulfur atom which links R and a perfluorovinyl group.

6. The process of claim 5 wherein R has more than one aromatic ring.

7. The process of claim 5 or 6 wherein R is 4,4'-bi-phenylene; phenylene; 9,9-diphenylfluorene; oxydiphenylene; thiodiphenylene; 2,2-diphenylenepropane; 1,1,1,3,3,3-hexafluoro-2,2-diphenylenepropane; 1,1-diphenylene-1-phenylethane 1,1,1-triphenyleneethane; 1,3,5-triphenylenebenzene; 1,3,5-(2-phenylene-2-propyl)benzene; 1,1,1-triphenylenemethane; 1,1,2,2-tetraphenylene-1,2-diphenylethane; bis(1,1-diphenyleneethyl)benzene; 1-(2-phenylene-2-propyl)-4-(1,1,-diphenyleneethyl)benzene; 2,2-diphenylene propane; 2,2'-diphenylene, 1,1,1,3,3,3-hexafluoropropane; 1,1-diphenylene-1-phenylethane; naphthalene; or anthracene.

**8.** The process of claim 4 wherein the monomer is an oligomer having perfluorovinyl end groups.

**9.** The process of claim 8 wherein the oligomer is a polyether; poly(carboxylic acid derivative); polysulfone; polycarbonate; polyimide; polyamide; polyamide-polyimides; liquid crystal polymer or mixtures thereof.

**10.** The process of claim 9 wherein the perfluorovinyl group is a perfluorovinyl aromatic ether.

**11.** The process of claim 4 wherein the polymer is linear.

**12.** The process of claim 11 wherein the polymer has a molecular weight of at least 10,000.

**13.** The process of claim 4 wherein m is 1.

**14.** The process of claim 4 wherein at least for 0.5 mole percent of the monomers m is 2 or 3.

**15.** The process of claim 14 wherein for 75 to 100 mole percent of the monomers m is 2 or 3.

**16.** The process of claim 14 wherein the monomers are heated to a first temperature to increase viscosity of the monomers, then heated to a second higher temperature to result in a crosslinked polymer.

**17.** The process of claim 16 wherein the first temperature is from 50°C to 400°C and the second temperature is from 100°C to 450°C.

**18.** A process for preparing a polymer having a backbone comprising aromatic hydrocarbyl groups, perfluorocyclobutane rings and non-carbon atoms by:
(a) contacting monomers having two dimerizable perfluorovinyl groups;
(b) exposing the monomers to sufficient heat of from 50°C to 400°C such that a polymer containing perfluorocyclobutane rings is formed thermally,
(c) exposing the polymer to sufficient heat from 100°C to 450°C such that crosslinking occurs and a crosslinked polymer is produced.

**19.** The process of claim 18 wherein the crosslinking takes place substantially without addition of a crosslinking agent or catalyst.

**20.** The process of claim 18 wherein the crosslinking step takes place at a temperature at least 50°C higher than the temperature used in step (b).

**21.** The process of claim 18 wherein the heat initiating crosslinking in step (c) is radiant heat.

**22.** The process of claim 18 wherein the crosslinking step (c) takes place in a shaping apparatus.

**23.** The process of claim 18 wherein the monomers have a structure represented by Formula I':

$CF_2 = CF\text{-}X\text{-}R\text{-}X\text{-}CF = CF_2$

wherein R represents an unsubstituted or inertly substituted aromatic hydrocarbyl group which reacts with perfluorovinyl groups residual in a substantially linear polymer to form a crosslinked or branched molecular structure; and each X is independently selected from the group consisting of groups having at least one non-carbon atom between R and $-CF = CF_2$.

**24.** The process of claim 23 wherein each X is independently an oxygen atom, a sulfur atom, a sulfoxide group, a sulfone group, a carbonyl group, and a thiocarbonyl group or a silanediyl group.

**Patentansprüche**

**1.** Polymer mit einer aromatische Kohlenwasserstoffgruppen, Perfluorcyclobutanringe und Nichtkohlenstoffatome enthaltenden Rückgratkette.

2. Polymer nach Anspruch 1,
**dadurch gekennzeichnet**,
daß die Nichtkohlenstoffatome Sauerstoff oder Schwefel sind.

3. Polymer nach Anspruch 1,
**dadurch gekennzeichnet**,
daß es eine Tg von mindestens 25 °C aufweist.

4. Verfahren zum Herstellen eines Polymeren, das eine aromatische Kohlenwasserstoffgruppen, Perfluorcyclobutanringe und Nichtkohlenstoffatome enthaltende Rückgratkette aufweist durch
(a) Inberührungbringen von Monomeren, die mindestens zwei dimerisierbare Perfluorvinylgruppen der Formel I aufweisen

$CF_2 = CF\text{-}X\text{-}R\text{-}(X\text{-}CF = CF_2)_m$

in der R eine nichtsubstituierte oder inert substituierte aromatische Kohlenwasserstoffgruppe ist, jedes X ist eine R und eine Perfluorvinylgruppe verbindende Gruppe und m ist eine ganze Zahl von 1 bis 3 und
(b) Aussetzen der Monomeren ausreichender Wärme von 40 °C bis 450 °C, so daß ein Perflourcyclobutanringe enthaltendes Polymer thermisch gebildet wird.

5. Verfahren nach Anspruch 4,
**dadurch gekennzeichnet**,
daß R 6 bis 50 Kohlenstoffatome aufweist und X eine Sauerstoff oder Schwefel enthaltende Gruppe ist, die R und eine Perfluorvinylgruppe verbindet.

6. Verfahren nach Anspruch 5,
**dadurch gekennzeichnet**,
daß R mehr als einen aromatischen Ring aufweist.

7. Verfahren nach Anspruch 5 oder 6,
**dadurch gekennzeichnet**,
daß R 4,4-Biphenylen, Phenylen, 9,9-Diphenylfluoren, Oxydiphenylen, Thiodiphenylen, 2,2-Diphenylenpropan, 1,1,1,3,3,3-Hexafluor-2,2-diphenylenpropan, 1,1-Diphenylen-1-Ppenylethan,1,1,1-Triphenylenethan, 1,3,5-Triphenylenbenzol, 1,3,5-(2-Phenylen-2-propyl)-benzol, 1,1,1-Triphenylenmethan, 1,1,2,2-Tetraphenylen-1,2-diphenylethan, Bis(1,1-diphenylenethyl)benzol, 1-(2-Phenylen-2-propyl)-4-(1,1-Diphenylenethyl)benzol , 2,2-Diphenylenpropan, 2,2'-Diphenylen, 1,1,1,3,3,3-Hexafluorpropan, 1,1-Diphenylen-1-phenylethan, Naphthalen oder Anthracen ist.

8. Verfahren nach Anspruch 4,
**dadurch gekennzeichnet**,
daß das Monomer ein Oligomer mit Perfluorvinylendgruppen ist.

9. Verfahren nach Anspruch 8
**dadurch gekennzeichnet**,
daß das Oligomer ein Polyether, Poly(carbonsäurederivat), Polysulfon, Polycarbonat, Polyimid, Polyamid, Polyamid-Polyimide, Flüssigkristallpolymer oder Mischungen derselben ist.

10. Verfahren nach Anspruch 9,
**dadurch gekennzeichnet**,
daß die Perfluorvinylgruppe ein perfluorvinylaromatischer Ether ist.

11. Verfahren nach Anspruch 4,
**dadurch gekennzeichnet**,
daß das Polymer linear ist.

12. Verfahren nach Anspruch 11,
**dadurch gekennzeichnet**,

daß das Polymer ein Molekulargewicht von mindestens 10.000 aufweist.

**13.** Verfahren nach Anspruch 4,
**dadurch gekennzeichnet**,
daß m 1 ist.

**14.** Verfahren nach Anspruch 4,
**dadurch gekennzeichnet**,
daß mindestens bei 0,5 Molprozent der Monomeren m 2 oder 3 ist.

**15.** Verfahren nach Anspruch 14,
**dadurch gekennzeichnet**,
daß bei 75 bis 100 Molprozent der Monomeren m 2 oder 3 ist.

**16.** Verfahren nach Anspruch 14,
**dadurch gekennzeichnet**,
daß die Monomeren auf eine erste Temperatur erwärmt werden, um die Viskosität der Monomeren zu erhöhen und dann auf eine zweite höhere Temperatur erwärmt werden, um ein vernetzendes Polymer zu erhalten.

**17.** Verfahren nach Anspruch 16,
**dadurch gekennzeichnet**,
daß die erste Temperatur von 50 °C bis 400 °C beträgt und die zweite Temperatur von 100 °C bis 450 °C beträgt.

**18.** Verfahren zum Herstellen eines Polymeren, das eine aromatische Kohlenwasserstoffgruppe, Perfluorcyclobutanringe und Nichtkohlenstoffatome enthaltende Rückgratkette aufweist durch

(a) Inberührungbringen von Monomeren, die zwei dimerisierbare Perfluorvinylgruppen aufweisen,
(b) Aussetzen der Monomeren ausreichender Wärme von 50 °C bis 400 °C, so daß ein Perfluorcyclobutanringe enthaltendes Polymer thermisch gebildet wird,
(c) Aussetzen des Polymeren ausreichender Wärme von 100 °C bis 450 °C, so daß Vernetzung auftritt und vernetztes Polymer erhalten wird.

**19.** Verfahren nach Anspruch 18,
**dadurch gekennzeichnet**,
daß die Vernetzung im wesentlichen ohne Zusatz eines Vernetzungsmittels oder Katalysators stattfindet.

**20.** Verfahren nach Anspruch 18,
**dadurch gekennzeichnet**,
daß die Vernetzung bei einer mindestens 50 °C höheren Temperatur stattfindet als der in Schritt (b) verwendeten Temperatur.

**21.** Verfahren nach Anspruch 18,
**dadurch gekennzeichnet**,
daß die Vernetzung in Schritt (c) auslösende Wärme Strahlungswärme ist.

**22.** Verfahren nach Anspruch 18,
**dadurch gekennzeichnet**,
daß der Vernetzungsschritt (c) in einer Formgebungsvorrichtung stattfindet.

**23.** Verfahren nach Anspruch 18,
**dadurch gekennzeichnet**,
daß die Monomeren eine durch die Formel I' wiedergegebene Struktur aufweisen:

$CF_2 = CF\text{-}X\text{-}R\text{-}X\text{-}CF = CF_2$

in der R eine nichtsubstituierte oder inert substituierte aromatische Kohlenwasserstoffgruppe ist, die mit

den verbleibenden Perfluorvinylgruppen in einem im wesentlichen linearen Polymer reagiert, um eine vernetzte oder verzweigte Molekularstruktur auszubilden und jedes X unabhängig ausgewählt ist aus der Gruppe bestehend aus Resten, die mindestens ein Nichtkohlenstoffatom zwischen R und $-CF=CF_2$ aufweisen.

**24.** Verfahren nach Anspruch 23,
**dadurch gekennzeichnet**,
daß jedes X unabhängig ein Sauerstoffatom, ein Schwefelatom, eine Sulfoxidgruppe, eine Sulfongruppe, eine Carbonylgruppe, eine Thiocarbonylgruppe oder eine Silandiylgruppe ist.

**Revendications**

**1.** Polymère ayant un squelette comprenant des groupes hydrocarbyle aromatiques, des cycles perfluorocyclobutane et des atomes différents du carbone.

**2.** Polymère selon la revendication 1, dans lequel les atomes différents du carbone sont l'oxygène ou le soufre.

**3.** Polymère selon la revendication 1, qui a une température de transition vitreuse d'au moins 25 ° C.

**4.** Procédé de préparation d'un polymère ayant un squelette comprenant des groupes hydrocarbyle aromatiques, des cycles perfluorocyclobutane et des atomes différents du carbone, dans lequel :
(a) on met en contact des monomères ayant au moins deux groupes perfluorovinyle dimérisables représentés par la formule I :

$$CF_2=CF\text{-}X\text{-}R\text{-}(X\text{-}CF=CF_2)_m$$

dans laquelle R représente un groupe hydrocarbyle aromatique substitué par un radical inerte ou non-substitué, chaque X étant tout groupe qui relie R et un groupe perfluorovinyle, et m est un entier compris entre 1 et 3 ; et
(b) on expose les monomères à une chaleur suffisante comprise entre 40 ° C et 450 ° C pour qu'un polymère contenant des cycles perfluorocyclobutane se forme par voie thermique.

**5.** Procédé selon la revendication 4, dans lequel R comporte 6 à 50 atomes de carbone et X est un groupe contenant un atome d'oxygène ou un atome de soufre, qui relie R et le groupe perfluorovinyle.

**6.** Procédé selon la revendication 5, dans lequel R comporte plus d'un cycle aromatique.

**7.** Procédé selon l'une des revendications 5 ou 6, dans lequel R est le 4,4'-biphénylène ; le phénylène ; le 9,9-diphénylfluorène; l'oxydiphénylène ; le thiodiphénylène ; le 2,2-diphénylènepropane ; le 1,1,1,3,3,3-hexafluoro-2,2-diphénylènepropane ; le 1,1-diphénylène-1-phényléthane ; le 1,1,1-triphénylèneéthane ; le 1,3,5-triphénylènebenzène ; le 1,3,5-(2-phénylène-2-propyl)benzène ; le 1,1,1-triphénylèneméthane ; le 1,1,2,2-tétraphénylène-1,2-diphényléthane ; le bis(1,1-diphénylèneéthyl)benzène ; le 1-(2-phénylène-2-propyl)-4-(1,1diphénylèneéthyl)benzène ; le 2,2-diphénylène propane ; le 2,2'-diphénylène, le 1,1,1,3,3,3-hexafluoropropane ; le 1,1-diphénylène-1-phényléthane ;le naphthalène ; ou l'anthracène.

**8.** Procédé selon la revendication 4, dans lequel le monomère est un oligomère ayant des groupes perfluorovinyle terminaux.

**9.** Procédé selon la revendication 8, dans lequel l'oligomère est un polyéther ; un dérivé d'acide carboxylique polymère ; une polysulfone ; un polycarbonate ; un polyimide ; un polyamide ; des polyamide-polyimides ; un polymère sous forme de cristal liquide ou leurs mélanges.

**10.** Procédé selon la revendication 9, dans lequel le groupe perfluorovinyle est un éther aromatique de perfluorovinyle.

**11.** Procédé selon la revendication 4, dans lequel le polymère est linéaire.

**12.** Procédé selon la revendication 11, dans lequel le polymère a un poids moléculaire d'au moins 10 000.

**13.** Procédé selon la revendication 4, dans lequel m est 1.

**14.** Procédé selon la revendication 4, dans lequel pour au moins 0,5 mode pour-cent des monomères, m est 2 ou 3.

**15.** Procédé selon la revendication 14, dans lequel pour 75 à 100 mode pour-cent des monomères, m est 2 ou 3.

**16.** Procédé selon la revendication 14, dans lequel les monomères sont chauffés à une première température pour augmenter la viscosité des monomères, puis chauffés à une seconde température plus élevée pour obtenir la réticulation du polymère.

**17.** Procédé selon la revendication 16, dans lequel la première température est de 50 °C à 400 °C et la seconde température est de 100 °C à 450 °C.

**18.** Procédé de préparation d'un polymère ayant un squelette comprenant des groupes hydrocarbyle aromatiques, des cycles perfluorocyclobutane et des atomes différents du carbone dans lequel :
(a) on met en contact des monomères ayant deux groupes perfluorovinyle dimérisables ;
(b) on expose les monomères à une chaleur de 50 °C à 400 °C suffisante pour qu'un polymère contenant des cycles perfluorocyclobutane soit formé par voie thermique ;
(c) on expose le polymère à une chaleur de 100 °C à 450 °C suffisante pour que la réticulation ait lieu et qu'un polymère réticulé soit produit.

**19.** Procédé selon la revendication 18, dans lequel la réticulation se fait essentiellement sans addition d'agent de réticulation ou de catalyseur.

**20.** Procédé selon la revendication 18, dans lequel le stade de réticulation est effectué à une température supérieure d'au moins 50 °C à la température utilisée au stade (b).

**21.** Procédé selon la revendication 18, dans lequel la chaleur amorçant la réticulation au stade (c) est une chaleur de rayonnement.

**22.** Procédé selon la revendication 18, dans lequel le stade de réticulation (c) a lieu dans un appareil de mise en forme.

**23.** Procédé selon la revendication 18, dans lequel les monomères ont une structure représentée par la formule I' :

$$CF_2=CF\text{-}X\text{-}R\text{-}X\text{-}CF=CF_2$$

dans laquelle R représente un groupe hydrocarbyle aromatique non substitué ou substitué par un radical inerte, qui réagit avec des groupes perfluorovinyle résiduaires dans un polymère substantiellement linéaire pour former une structure moléculaire réticulée ou ramifiée ; et chaque X est indépendamment choisi dans le groupe formé par les groupes ayant au moins un atome différent du carbone entre R et $-CF=CF_2$.

**24.** Procédé selon la revendication 23, dans lequel chaque X est indépendamment un atome d'oxygène, un atome de soufre, un groupe sulfoxyde, un groupe sulfone, un groupe carbonyle, et un groupe thiocarbonyle ou un groupe silanediyl.